# EUROPEAN PATENT SPECIFICATION

(11) **EP 1 720 892 B1**
(45) Date of publication and mention of the grant of the patent: **24.07.2013**
(21) Application number: 05722567.4
(22) Date of filing: 26.01.2005
(51) Int. Cl.: C07K 1/107, C07H 21/02, C07H 1/00, C07H 15/08, C07H 15/12

(54) **Branched polymer-modified sugars and nucleotides**
Mit verzweigten Polymeren modifizierte Zucker und Nukleotide
Sucres et nucleotides modifiés avec des polymères ramifiés

(30) Priority: 26.01.2004 US 539387 P; 12.02.2004 US 544411 P; 20.02.2004 US 546631 P; 22.03.2004 US 555504 P; 12.05.2004 US 570891 P; 23.07.2004 US 590649 P; 23.07.2004 US 590573 P; 29.07.2004 US 592744 P; 20.09.2004 US 611790 P; 29.09.2004 US 614518 P; 29.10.2004 US 623387 P; 09.11.2004 US 626678 P; 24.11.2004 US 997405; 24.11.2004 WO PCT/US2004/039712; 03.12.2004 WO PCT/US2004/040709; 06.01.2005 US 641956 P; 10.01.2005 US 643437 P; 10.01.2005 WO PCT/US2005/000799
(43) Date of publication of application: 15.11.2006
(73) Proprietor: BioGeneriX AG, 68199 Mannheim (DE)
(72) Inventor: DEFREES, Shawn, North Wales, PA 19454 (US); BOWE, Caryn, Doylestown, PA 18901 (US)
(74) Representative: Engelhard, Markus
(86) International application number: PCT/US2005/002522
(87) International publication number: WO 2005/072371

(56) References cited:
- WO-A-03/031464
- US-A- 5 405 753
- US-A- 5 932 462
- US-A- 6 113 906
- US-B1- 6 399 336

## Description

### BACKGROUND OF THE INVENTION

### Field of the Invention

The present invention resides in the field of modified sugars and nucleotides thereof.

### Background

Post-expression *in vitro* modification of peptides is an attractive strategy to remedy the deficiencies of methods that rely on controlling glycosylation by engineering expression systems; including both modification of glycan structures or introduction of glycans at novel sites. A comprehensive toolbox of recombinant eukaryotic glycosyltransferases is becoming available, making *in vitro* enzymatic synthesis of mammalian glycoconjugates with custom designed glycosylation patterns and glycosyl structures possible. See, for example, U.S. Patent No. 5,876,980; 6,030,815; 5,728,554; 5,922,577; and WO/9831826; US2003180835; and WO 03/031464.

Enzyme-based syntheses have the advantages of regioselectivity and stereoselectivity. Moreover, enzymatic syntheses are performed using unprotected substrates. Three principal classes of enzymes are used in the synthesis of carbohydrates, glycosyltransferases (*e.g.,* sialyltransferases, oligosaccharyltransferases, N-acetylglucosaminyltransferases), and glycosidases. The glycosidases are further classified as exoglycosidases (*e.g.*, β-mannosidase, β-glucosidase), and endoglycosidases (*e.g.*, Endo-A, Endo-M). Each of these classes of enzymes has been successfully used synthetically to prepare carbohydrates. For a general review, *see*, Crout et al., Curr. Opin. Chem. Biol. 2: 98-111 (1998).

Glycosyltransferases modify the oligosaccharide structures on glycopeptides. Glycosyltransferases are effective for producing specific products with good stereochemical and regiochemical control. Glycosyltransferases have been used to prepare oligosaccharides and to modify terminal N- and O-linked carbohydrate structures, particularly on glycopeptides produced in mammalian cells. For example, the terminal oligosaccharides of glycopeptides have been completely sialylated and/or fucosylated to provide more consistent sugar structures, which improves glycopeptide pharmacodynamics and a variety of other biological properties. For example, β-1,4-galactosyltransferase was used to synthesize lactosamine, an illustration of the utility of glycosyltransferases in the synthesis of carbohydrates (*see, e.g.*, Wong et al., J. Org. Chem. 47: 5416-5418 (1982)). Moreover, numerous synthetic procedures have made use of α-sialyltransferases to transfer sialic acid from cytidine-5'-monophospho-N-acetylneuraminic acid to the 3-OH or 6-OH of galactose (*see, e.g.*, Kevin et al., Chem. Eur. J. 2: 1359-1362 (1996)). Fucosyltransferases are used in synthetic pathways to transfer a fucose unit from guanosine-5'-diphosphofucose to a specific hydroxyl of a saccharide acceptor. For example, Ichikawa prepared sialyl Lewis-X by a method that involves the fucosylation of sialylated lactosamine with a cloned fucosyltransferase (Ichikawa et al., J. Am. Chem. Soc. 114: 9283-9298 (1992)). For a discussion of recent advances in glycoconjugate synthesis for therapeutic use *see*, Koeller et al., Nature Biotechnology 18: 835-841 (2000). *See* also, U.S. Patent No. 5,876,980; 6,030,815; 5,728,554; 5,922,577; and WO/9831826.

In addition to manipulating the structure of glycosyl groups on polypeptides, interest has developed in preparing glycopeptides that are modified with one or more non-saccharide modifying group, such as water soluble polymers. Poly(ethyleneglycol) ("PEG") is an exemplary polymer that has been conjugated to polypeptides. The use of PEG to derivatize peptide therapeutics has been demonstrated to reduce the immunogenicity of the peptides. For example, U.S. Pat. No. 4,179,337 (Davis et al.) discloses non-immunogenic polypeptides, such as enzymes and peptide hormones coupled to polyethylene glycol (PEG) or polypropylene glycol. Between 10 and 100 moles of polymer are used per mole polypeptide: Although the in vivo clearance time of the conjugate is prolonged relative to that of the polypeptide, only about 15% of the physiological activity is maintained. Thus, the prolonged circulation half-life is counterbalanced by the dramatic reduction in peptide potency.

The loss of peptide activity is directly attributable to the non-selective nature of the chemistries utilized to conjugate the water-soluble polymer. The principal mode of attachment of PEG, and its derivatives, to peptides is a non-specific bonding through a peptide amino acid residue. For example, U.S. Patent No. 4,088,538 discloses an enzymatically active polymer-enzyme conjugate of an enzyme covalently bound to PEG. Similarly, U.S. Patent No. 4,496,689 discloses a covalently attached complex of a 1 proteinase inhibitor with a polymer such as PEG. Abuchowski et al. (J. Biol. Chem. 252: 3578 (1977) discloses the covalent attachment of MPEG to an amine group of bovine serum albumin. U.S. Patent No. 4,414,147 discloses a method of rendering interferon less hydrophobic by conjugating it to an anhydride of a dicarboxylic acid, such as poly(ethylene succinic anhydride). PCT WO 87/00056 discloses conjugation of PEG and poly(oxyethylated) polyols to such proteins as interferon-β, interleukin-2 and immunotoxins. EP 154,316 discloses and claims chemically modified lymphokines, such as IL-2 containing PEG bonded directly to at least one primary amino group of the lymphokine. U.S. Patent No. 4,055,635 discloses pharmaceutical compositions of a water-soluble complex of a proteolytic enzyme linked covalently to a polymeric substance such as a polysaccharide.

Another mode of attaching PEG to peptides is through the non-specific oxidation of glycosyl residues on a glycopeptide. The oxidized sugar is utilized as a locus for attaching a PEG moiety to the peptide. For example M'Timkulu (WO 94/05332) discloses the use of an amino-PEG to add PEG to a glycoprotein. The glycosyl moieties are randomly oxidized to the corresponding aldehydes, which are subsequently coupled to the amino-PEG.

In each of the methods described above, poly(ethyleneglycol) is added in a random, non-specific manner to reactive residues on a peptide backbone. For the production of therapeutic peptides, it is clearly desirable to utilize a derivitization strategy that results in the formation of a specifically labeled, readily characterizable, essentially homogeneous product. A promising route to preparing specifically labeled peptides is through the use of enzymes, such as glycosyltransferases to append a modified sugar moiety onto a peptide. The modified sugar moiety must function as a substrate for the glycosyltransferase and be appropriately activated. Hence, synthetic routes that provide facile access to activated modified sugars are desirable. The present invention provides such a route.

### SUMMARY OF THE INVENTION

The present invention provides for compounds in accordance with the appended claims.

The present invention provides polymeric species, sugars and activated sugars conjugated to these polymeric species and nucleotide sugars that include these polymers. The polymers are branched-chain polymers.

The polymeric modifying group can be attached at any position of the sugar moiety. The invention is exemplified by reference to an embodiment in which the polymeric modifying group is attached to C-5 of a furanose or C-6 of a pyranose.

The invention provides a sugar or a sugar nucleotide that is conjugated to a polymer in accordance with the appended claims:

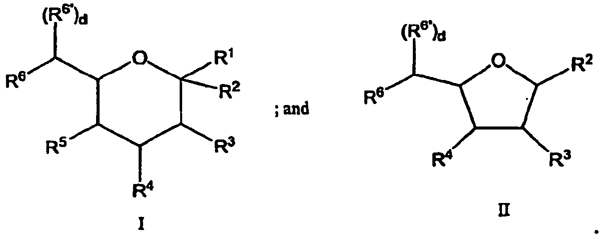

In Formulae I and II, R¹ is H, CH₂OR⁷, COOR⁷ or OR⁷, in which R⁷ represents H, substituted or unsubstituted alkyl or substituted or unsubstituted heteroalkyl. R² is H, OH or a moiety that includes a nucleotide. An exemplary R² species according to this embodiment has the formula: 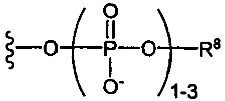 in which R⁸ is a nucleoside.

The symbols R³, R⁴, R⁵, R⁶ and R^{6'} independently represent H, substituted or unsubstituted alkyl, OR⁹, NHC(O)R¹⁰. The index d is 0 or 1. R⁹ and R¹⁰ are independently selected from H, substituted or unsubstituted alkyl, substituted or unsubstituted heteroalkyl or sialic acid. At least one of R³, R⁴, R⁵, R⁶ or R^{6'} includes the polymeric modifying moiety *e.g.*, PEG. In an exemplary embodiment, R⁶ and R^{6'}, together with the carbon to which they are attached are components of the side chain of sialic acid. In a further exemplary embodiment, this side chain is functionalized with the polymeric modifying moiety.

Discussed herein is an embodiment wherein the polymeric moiety is bound to the sugar core, generally through a heteroatom on the core, through a linker, L, as shown below: 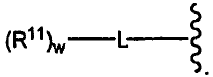 R¹¹ is the polymeric moiety and L is selected from a bond and a linking group. The index w represents and integer selected from 1-6, preferably 1-3 and more preferably 1-2. Exemplary linking groups include substituted or unsubstituted alkyl, substituted or unsubstituted heteroalkyl moieties and sialic acid. An exemplary component of the linker is an acyl moiety.

When L is a bond it is formed between a reactive functional group on a precursor of R¹¹ and a reactive functional group of complementary reactivity on a precursor of L. L can be in place on the saccharide core prior to reaction with R¹¹. Alternatively, R¹¹ and L can be incorporated into a preformed cassette that is subsequently attached to the saccharide core. As set forth herein, the selection and preparation of precursors with appropriate reactive functional groups is within the ability of those skilled in the art. Moreover, coupling the precursors proceeds by chemistries that are well understood in the art.

In an exemplary embodiment L is a linking group that is formed from an amino acid, providing a modified sugar in which the polymeric modifying moiety is attached through a substituted alkyl linker. An exemplary linker is glycine.

In an exemplary embodiment, R⁶ includes the polymeric modifying moiety. In another exemplary embodiment, R⁶ includes both the polymeric modifying moiety and a linker, L, joining the modifying moiety to the remainder of the molecule.

In the present invention, the polymeric modifying moiety is a branched structure that includes two or more polymeric chains attached to central moiety. An exemplary structure of a useful polymeric modifying moiety precursor according to this embodiment of the invention has the formula: 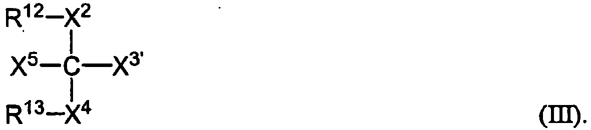 The sugars and nucleotide sugars according to this formula are essentially pure water-soluble polymers. X^{3'} is a moiety that includes an ionizable (e.g., COOH) or other reactive functional group, *see*, e.g., *infra*. C is carbon. X⁵ is a non-reactive group (e.g., H, unsubstitited alkyl, unsubstituted heteroalkyl). R¹² and R¹³ are independently selected polymeric arms, e.g., nonpeptidic, nonreactive polymeric arms. X² and X⁴ are linkage fragments that are preferably essentially non-reactive under physiological conditions, which may be the same or different such that they form the compounds of the claims. When X^{3'} is reacted with a reactive functional group of complementary reactivity on a linker, or linker-sugar cassette X^{3'} is converted to a component of linkage fragment X³.

By reaction of the precursor with a suitable sugar or sugar linker species the invention provides sugars and nucleotide sugars that have the formulae: 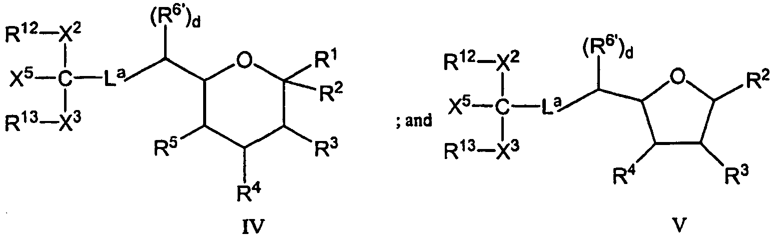 in which the identity of the radicals represented by the various symbols is the same as that discussed hereinabove. L^{a} is a substituted or unsubstituted alkyl or substituted or unsubstituted heteroalkyl moiety. In an exemplary embodiment, L^{a} is a moiety of the side chain of sialic acid that is functionalized with the polymeric modifying moiety as shown.

The polymeric modifying moiety is described in the claims and comprises two or more repeating units that can be water-soluble. Exemplary water-soluble polymers of use in the compounds of the invention are PEG, e.g., m-PEG

The sugar moiety of the polymeric conjugates of the invention is selected from both natural and unnatural furanoses and hexanoses. The unnatural saccharides optionally include an alkylated or acylated hydroxyl and/or amine moiety, e.g., ethers, esters and amide substituents on the ring. Other unnatural saccharides include an H, hydroxyl, ether, ester or amide substituent at a position on the ring at which such a substituent is not present in the natural saccharide. Alternatively, the carbohydrate is missing a substituent that would be found in the carbohydrate from which its name is derived, e.g., deoxy sugars. Still further exemplary unnatural sugars include both oxidized (e.g., -onic and uronic acids) and reduced (sugar alcohols) carbohydrates. The sugar moiety can be a mono-, oligo- or poly-saccharide.

Exemplary natural sugars of use in the present invention include glucose, glucosamine, galactose, galactosamine, fucose, mannose, mannosamine, xylanose, ribose, N-acetyl glucose, N-acetyl glucosamine, N-acetyl galactose, N-acetyl galactosamine, and sialic acid.

An exemplary sialic acid-based conjugate has the formula: 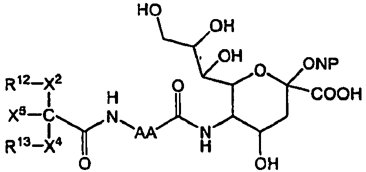 in which AA is that portion of an amino acid residue that does not include the carboxyl moiety and NP is a nucleotide phosphate. ONP can also be replaced by an activating moiety to form an activated sugar. As will be appreciated by those of skill in the art, the polymeric modifying moiety-linker can also be attached to the sialic acid side chain at C-6, C-7 and/or C-9.

Also provided is a synthetic method for producing an activated sialic acid-PEG conjugate that is an appropriate substrate for an enzyme, e.g., a glycosyltransferase. The method includes the steps: (a) contacting mannosamine with an activated, N-protected amino acid (or an amino acid functionalized with a polymeric modifying moiety, a linker precursor or a linker-polymeric modifying moiety cassette) under conditions appropriate to form an amide conjugate between the mannosamine and the N-protected amino acid; (b) contacting the amide conjugate with pyruvate and sialic acid aldolase under conditions appropriate to convert the amide conjugate to a sialic acid amide conjugate; (c) contacting the sialic acid amide conjugate with cytidine triphosphates, and a synthetase under conditions appropriate to form a cytidine monophosphate sialic acid amide conjugate; (d) removing the N-protecting group from the cytidine monophosphate sialic acid amide conjugate, thereby producing a free amine; and (e) contacting the free amine with an activated branched PEG, thereby forming the cytidine monophosphate sialic acid-poly(ethylene glycol).

The nucleoside can be selected from both natural and unnatural nucleosides. Exemplary natural nucleosides of use in the present invention include cytosine, thymine, guanine, adenine and uracil. The art is replete with structures of unnatural nucleosides and methods of making them.

Exemplary modified sugar nucleotides of the invention include polymerically-modified GDP-Man, GDP-Fuc, UDP-Gal, UDP-GalNAc, UDP-Glc, UDP-GlcNAc, UDP-Glc, UDP-GlcUA and CMP-SA and the like. Examples include UDP-Gal-2'-NH-PEG, UDP-Glc-2'-NH-PEG, CMP-5'-PEG-SA and the like. Compounds encompassed by the invention include those in which the L-R¹¹ moiety is conjugated to a furanose or a pyranose, e.g., at C-5 of a furanose or at C-6 of a pyranose, generally through a heteroatom attached to this carbon atom.

When the compound of the invention is a nucleotide sugar, or activated sugar, the polymeric conjugates of the nucleotide sugars are generally substrates for an enzyme that transfers the sugar moiety and its polymeric substituent onto an appropriate acceptor moiety of a substrate. Accordingly, the invention also provides substrates modified by glycoconjugation using a polymeric conjugate of a nucleotide sugar, or activated sugar, and an appropriate enzyme. Substrates that can be glycoconjugated using a compound of the invention include peptides, e.g., glycopeptides, peptides, lipids, e.g., glycolipids and aglycones (sphingosines, ceramides).

### BRIEF DESCRIPTION OF THE DRAWINGS

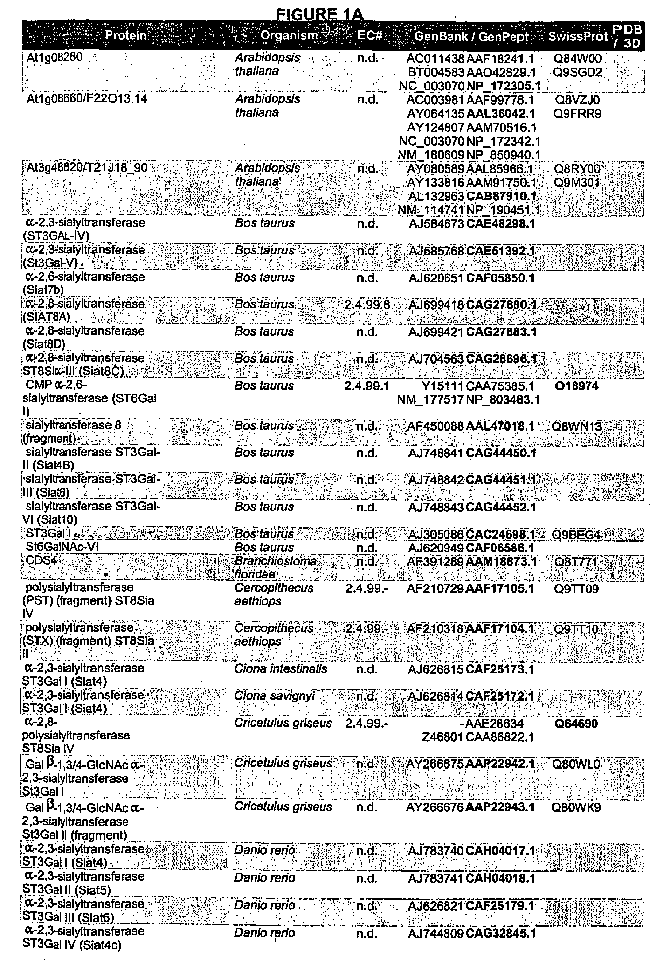

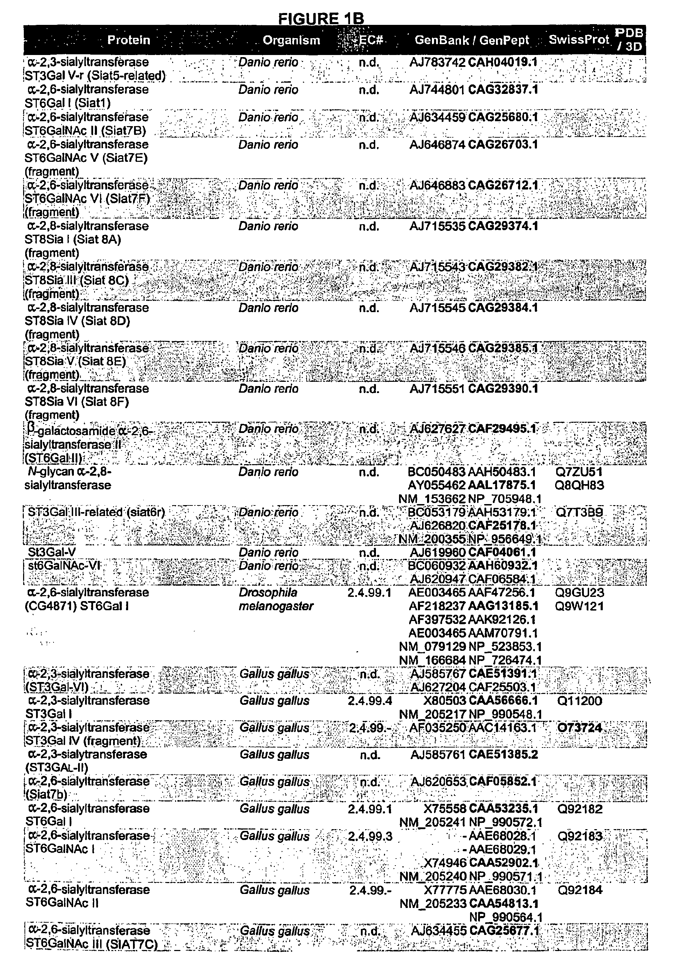

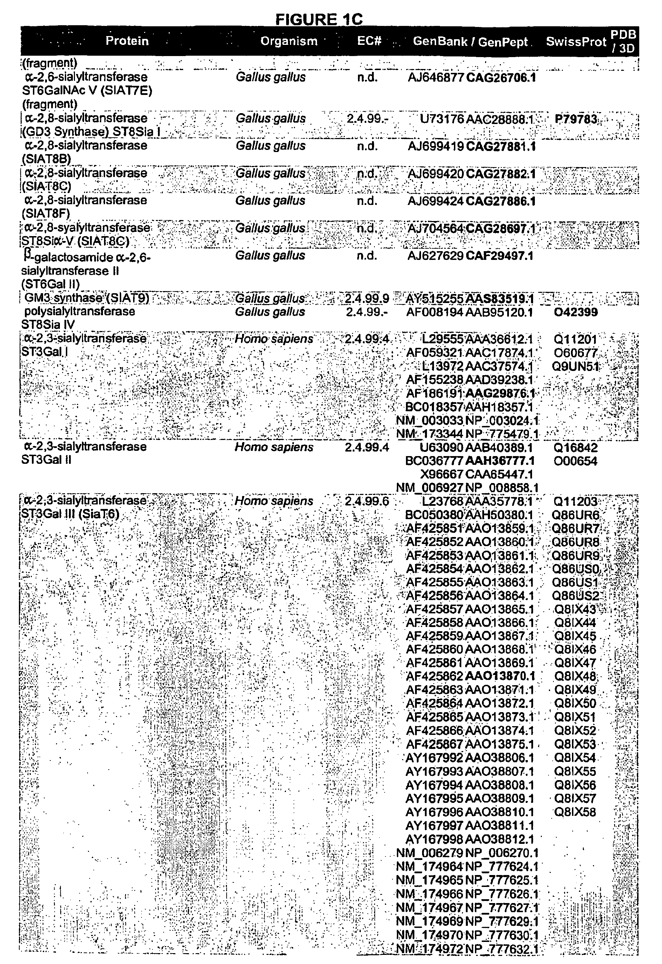

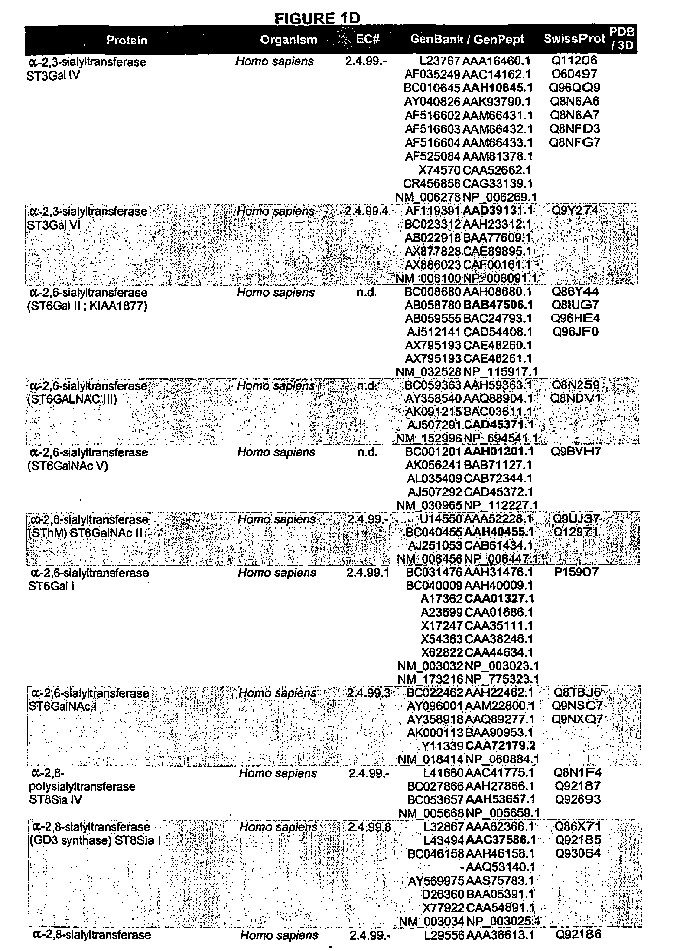

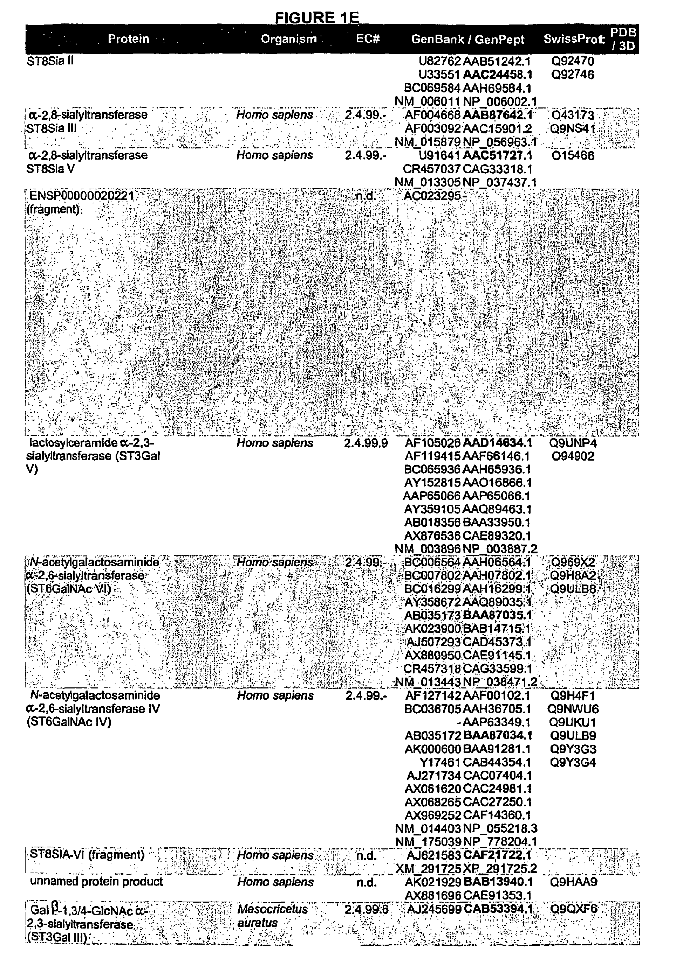

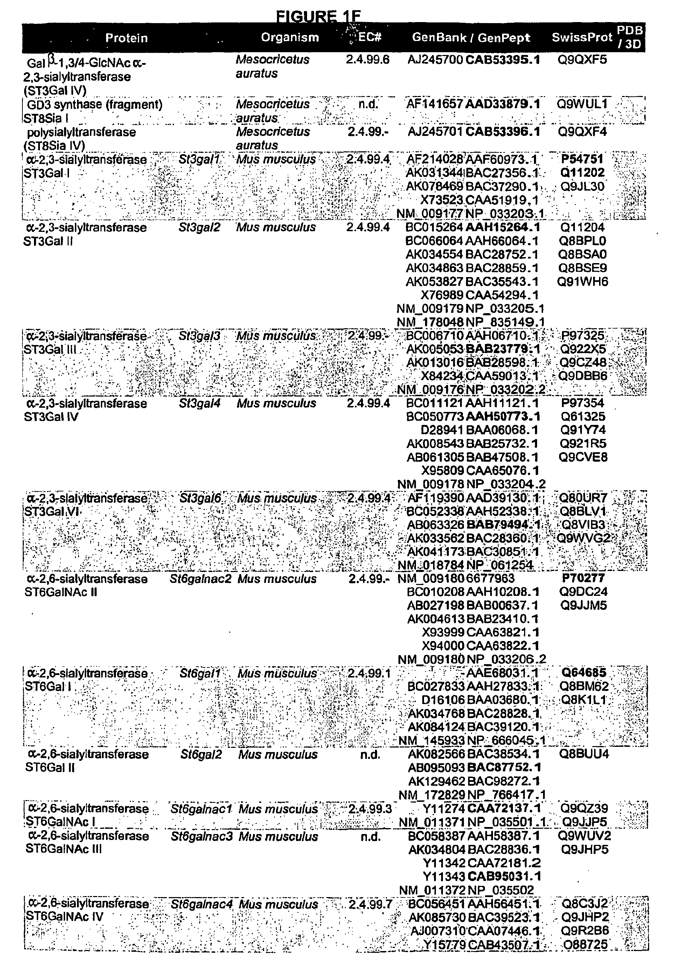

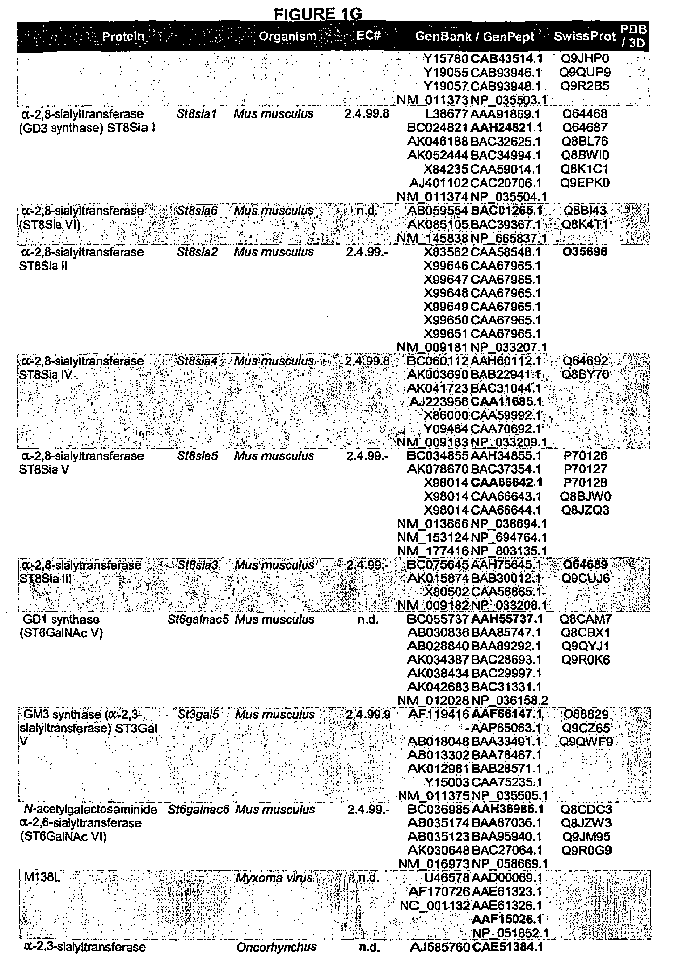

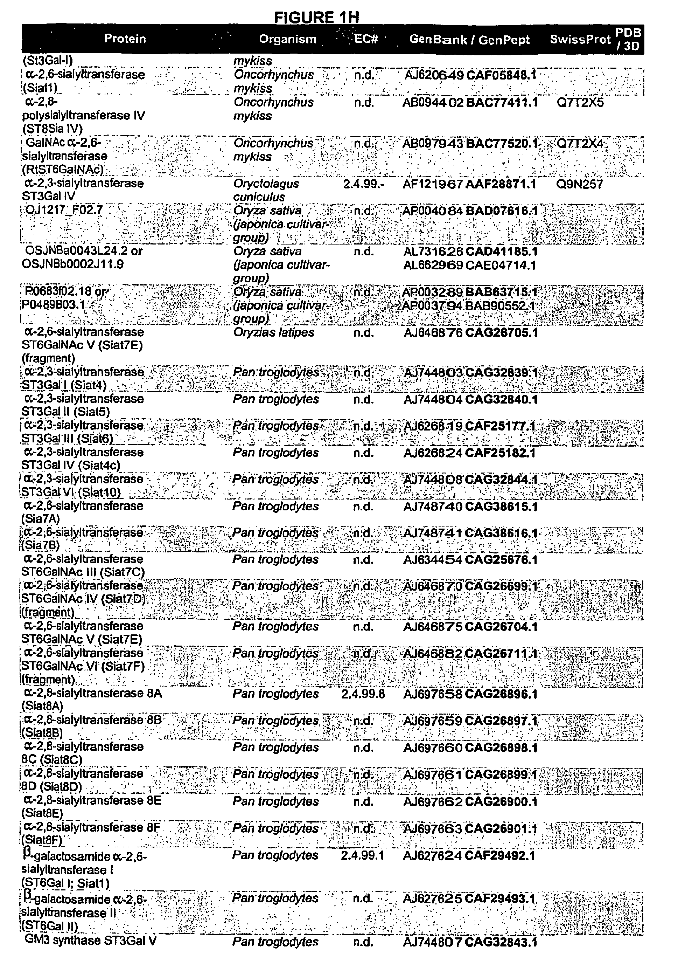

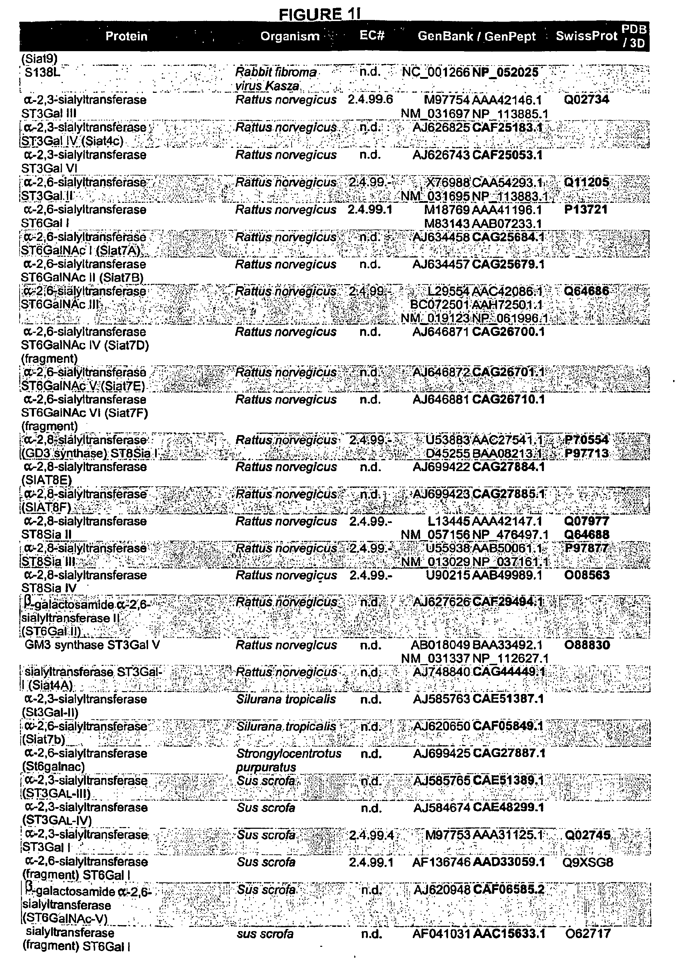

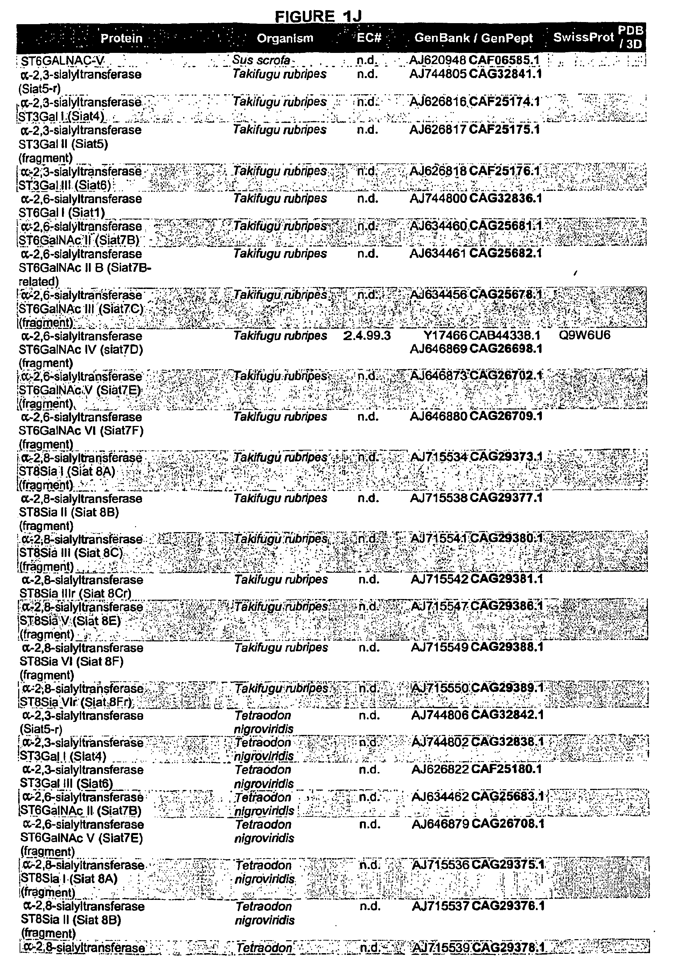

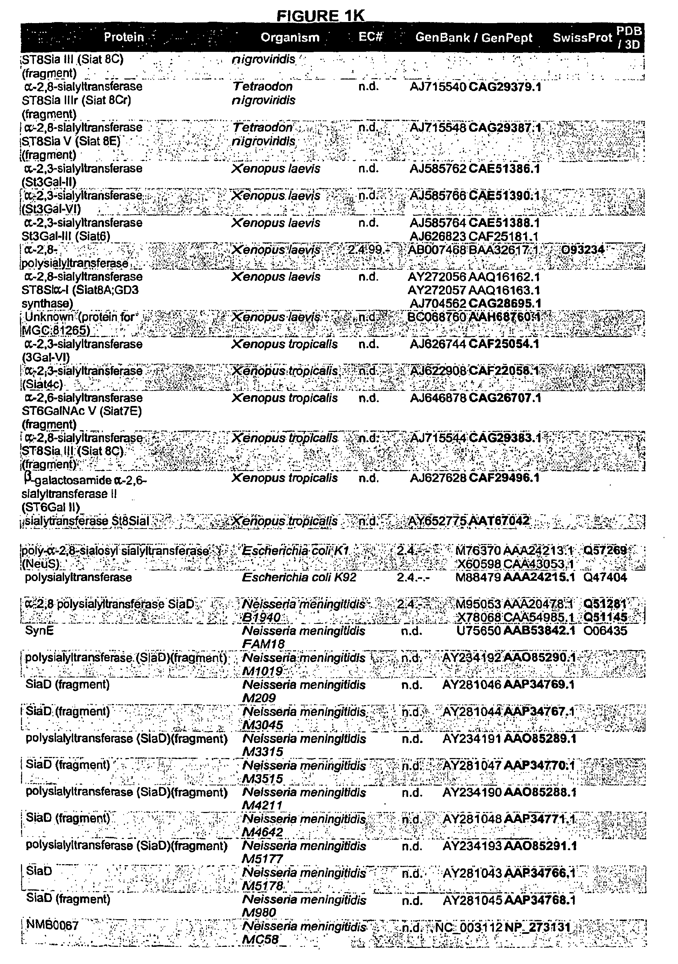

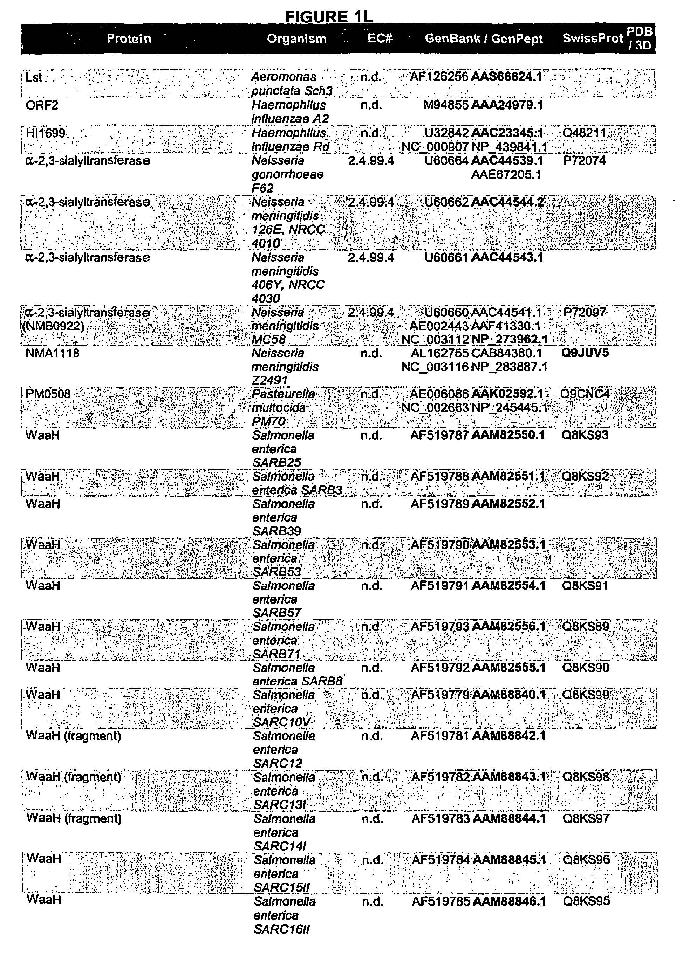

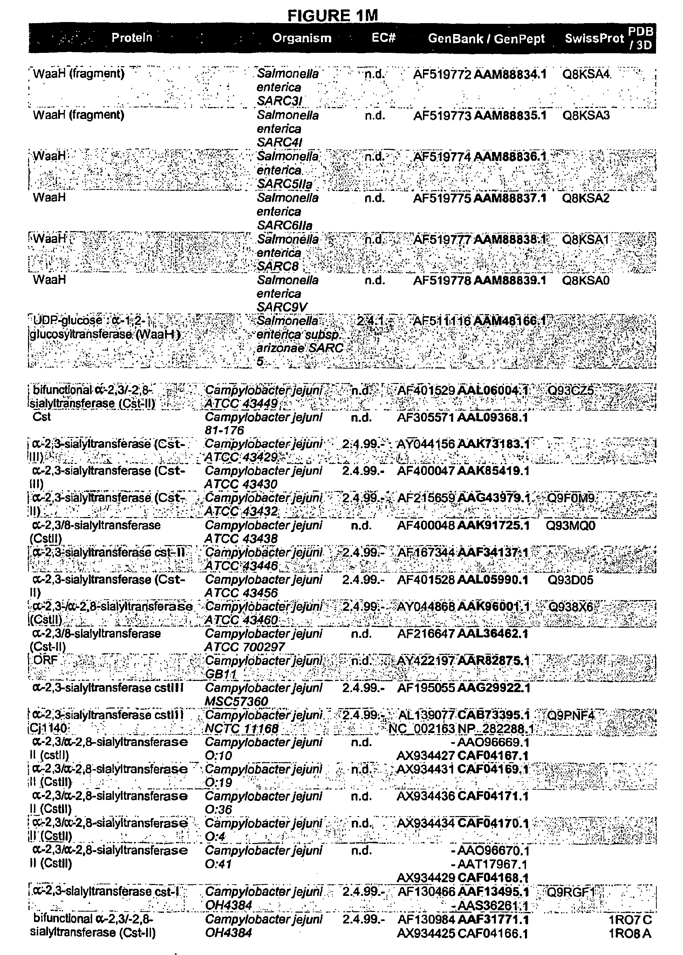

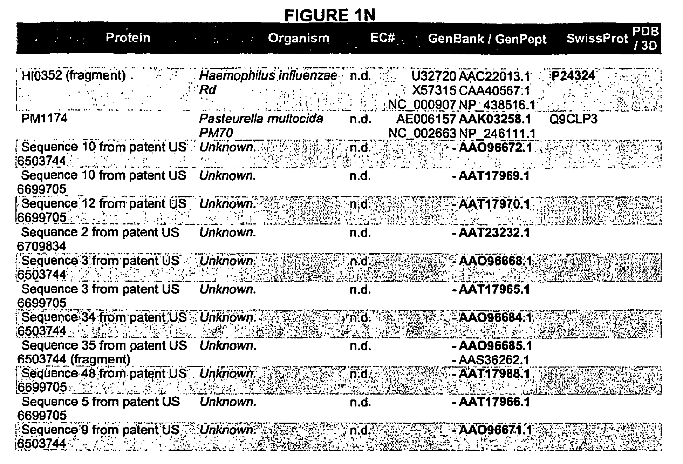

**FIG. 1** is a table of sialyltransferases for which selected modified sialic acid nucleotides and activated sugars are substrates.

**FIG. 2** is a general synthetic scheme of the invention for preparing a sialic acid-poly(ethylene glycol) conjugate.

**FIG. 3** is a synthetic scheme of the invention for preparing a sialic acid-glycyl-poly(ethylene glycol) conjugate.

### DETAILED DESCRIPTION OF THE INVENTION AND THE EMBODIMENTS

### Abbreviations

Branched and unbranched PEG, poly(ethyleneglycol), e.g., m-PEG, methoxy-poly(ethylene glycol); Branched and unbranched PPG, poly(propyleneglycol), e.g., m-PPG, methoxy-poly(propylene glycol); Fuc, fucosyl; Gal, galactosyl; GalNAc, N-acetylgalactosaminyl; Glc, glucosyl; GlcNAc, N-acetylglucosaminyl; Man, mannosyl; ManAc, mannosaminyl acetate; Sia, sialic acid; and NeuAc, N-acetylneuraminyl.

### Definitions

The term "sialic acid" refers to any member of a family of nine-carbon carboxylated sugars. The most common member of the sialic acid family is N-acetyl-neuraminic acid (2-keto-5-acetamido-3,5-dideoxy-D-glycero-D-galactononulopyranos-1-onic acid (often abbreviated as Neu5Ac, NeuAc, or NANA). A second member of the family is N-glycolyl-neuraminic acid (Neu5Gc or NeuGc), in which the N-acetyl group ofNeuAc is hydroxylated. A third sialic acid family member is 2-keto-3-deoxy-nonulosonic acid (KDN) (Nadano et al. (1986) J. Biol. Chem. 261: 11550-11557; Kanamori et al., J. Biol. Chem. 265: 21811-21819 (1990)). Also included are 9-substituted sialic acids such as a 9-O-C₁-C₆ acyl-Neu5Ac like 9-O-lactyl-Neu5Ac or 9-O-acetyl-Neu5Ac, 9-deoxy-9-fluoro-Neu5Ac and 9-azido-9-deoxy-Neu5Ac. For review of the sialic acid family, *see*, *e.g.*, Varki, Glycobiology 2: 25-40 (1992); Sialic Acids: Chemistry, Metabolism and Function, R. Schauer, Ed. (Springer-Verlag, New York (1992)). The synthesis and use of sialic acid compounds in a sialylation procedure is disclosed in international application WO 92/16640, published October 1, 1992.

As used herein, the term "modified sugar," refers to a naturally- or non-naturally-occurring carbohydrate that is enzymatically added onto an amino acid or a glycosyl residue of a peptide in a process of the invention. The modified sugar is selected from a number of enzyme substrates including, but not limited to sugar nucleotides (mono-, di-, and triphosphates), activated sugars (*e.g.*, glycosyl halides, glycosyl mesylates) and sugars that are neither activated nor nucleotides. The "modified sugar" is covalently functionalized with a "modifying group." Useful modifying groups include, but are not limited to, water-soluble polymers, therapeutic moieties, diagnostic moieties, biomolecules and the like. The modifying group is preferably not a naturally occurring, or an unmodified carbohydrate. The locus of functionalization with the modifying group is selected such that it does not prevent the "modified sugar" from being added enzymatically to a peptide.

The term "water-soluble" refers to moieties that have some detectable degree of solubility in water. Methods to detect and/or quantify water solubility are well known in the art. Exemplary water-soluble polymers include peptides, saccharides, poly(ethers), poly(amines), poly(carboxylic acids) and the like. Peptides can have mixed sequences or be composed of a single amino acid, *e.g.*, poly(lysine). An exemplary polysaccharide is poly(sialic acid). An exemplary poly(ether) is poly(ethylene glycol), *e.g.*, m-PEG. Poly(ethylene imine) is an exemplary polyamine, and poly(acrylic) acid is a representative poly(carboxylic acid). Exemplary polymers are typically comprised of 2-8 polymeric units.

The polymer backbone of the water-soluble polymer can be poly(ethylene glycol) (i.e. PEG). However, it should be understood that other related polymers are also suitable for use in the practice of this invention and that the use of the term PEG or poly(ethylene glycol) is intended to be inclusive and not exclusive in this respect. The term PEG includes poly(ethylene glycol) in any of its forms, including alkoxy PEG, difunctional PEG, multiarmed PEG, forked PEG, branched PEG, pendent PEG (i.e. PEG or related polymers having one or more functional groups pendent to the polymer backbone), or PEG with degradable linkages therein.

The polymer backbone is branched. Branched polymer backbones are generally known in the art. Typically, a branched polymer has a central branch core moiety and a plurality of linear polymer chains linked to the central branch core. PEG is commonly used in branched forms that can be prepared by addition of ethylene oxide to various polyols, such as glycerol, pentaerythritol and sorbitol. The branched PEG molecules of the invention are defined in the claims.

The term "glycoconjugation," as used herein, refers to the enzymatically mediated conjugation of a modified sugar species to an amino acid or glycosyl residue of a polypeptide, *e.g.*, a mutant human growth hormone of the present invention. A subgenus of "glycoconjugation" is "glycol-PEGylation," in which the modifying group of the modified sugar is poly(ethylene glycol), and alkyl derivative (*e.g.*, m-PEG) or reactive derivative (*e.g.*, H₂N-PEG, HOOC-PEG) thereof.

The term, "glycosyl linking group," as used herein refers to a glycosyl residue to which a modifying group (e.g., PEG moiety, therapeutic moiety, biomolecule) is covalently attached; the glycosyl linking group joins the modifying group to the remainder of the conjugate. In the methods of the invention, the "glycosyl linking group" becomes covalently attached to a glycosylated or unglycosylated peptide, thereby linking the agent to an amino acid and/or glycosyl residue on the peptide. A "glycosyl linking group" is generally derived from a "modified sugar" by the enzymatic attachment of the "modified sugar" to an amino acid and/or glycosyl residue of the peptide. The glycosyl linking group can be a saccharide-derived structure that is degraded during formation of modifying group-modified sugar cassette (e.g., oxidation→Schiff base formation→reduction), or the glycosyl linking group may be intact. An "intact glycosyl linking group" refers to a linking group that is derived from a glycosyl moiety in which the saccharide monomer that links the modifying group and to the remainder of the conjugate is not degraded, e.g., oxidized, e.g., by sodium metaperiodate. "Intact glycosyl linking groups" of the invention may be derived from a naturally occurring oligosaccharide by addition of glycosyl unit(s) or removal of one or more glycosyl unit from a parent saccharide structure.

Where substituent groups are specified by their conventional chemical formulae, written from left to right, they equally encompass the chemically identical substituents, which would result from writing the structure from right to left, *e.g.*, -CH₂O- is intended to also recite -OCH₂-.

The term "alkyl," by itself or as part of another substituent means, unless otherwise stated, a straight or branched chain, or cyclic hydrocarbon radical, or combination thereof, which may be fully saturated, mono- or polyunsaturated and can include di- and multivalent radicals, having the number of carbon atoms designated (*i.e.* C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl," unless otherwise noted, is also meant to include those derivatives of alkyl defined in more detail below, such as "heteroalkyl." Alkyl groups that are limited to hydrocarbon groups are termed "homoalkyl".

The term "alkylene" by itself or as part of another substituent means a divalent radical derived from an alkane, as exemplified, but not limited, by -CH₂CH₂CH₂CH₂-, and further includes those groups described below as "heteroalkylene." Typically, an alkyl (or alkylene) group will have from 1 to 24 carbon atoms, with those groups having 10 or fewer carbon atoms being preferred in the present invention. A "lower alkyl," or "lower alkylene" is a shorter chain alkyl or alkylene group, generally having eight or fewer carbon atoms.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The term "heteroalkyl," by itself or in combination with another term, means, unless otherwise stated, a stable straight or branched chain, or cyclic hydrocarbon radical, or combinations thereof, consisting of the stated number of carbon atoms and at least one heteroatom selected from the group consisting of O, N, Si and S, and wherein the nitrogen and sulfur atoms may optionally be oxidized and the nitrogen heteroatom may optionally be quaternized. The heteroatom(s) O, N and S and Si may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, and -CH=CH-N(CH₃)-CH₃- Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (*e.g.*, alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-.

The terms "cycloalkyl" and "heterocycloalkyl", by themselves or in combination with other terms, represent, unless otherwise stated, cyclic versions of "alkyl," and "heteroaryl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include, but not be limited to, trifluoromethyl, 2,2,2-trifluoroethyl, 4-chlorobutyl, 3-bromopropyl, and the like.

The term "aryl" means, unless otherwise stated, a polyunsaturated, aromatic, substituent that can be a single ring or multiple rings (preferably from 1 to 3 rings), which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, tetrazolyl, benzo[b]furanyl, benzo[b]thienyl, 2,3-dihydrobenzo[1,4]dioxin-6-yl, benzo[1,3]dioxol-5-yl and 6-quinolyl. Substituents for each of the above noted aryl and heteroaryl ring systems are selected from the group of acceptable substituents described below.

For brevity, the term "aryl" when used in combination with other terms (*e.g.*, aryloxy, arylthioxy, arylalkyl) includes both aryl and heteroaryl rings as defined above. Thus, the term "arylalkyl" is meant to include those radicals in which an aryl group is attached to an alkyl group (*e.g.*, benzyl, phenethyl, pyridylmethyl and the like) including those alkyl groups in which a carbon atom (*e.g.*, a methylene group) has been replaced by, for example, an oxygen atom (*e.g.*, phenoxymethyl, 2-pyridyloxymethyl, 3-(1-naphthyloxy)propyl, and the like).

Each of the above terms (*e.g.*, "alkyl," "heteroalkyl," "aryl" and "heteroaryl") is meant to include both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generically referred to as "alkyl group substituents," and they can be one or more of a variety of groups selected from, but not limited to: -OR', =O, =NR', =N-OR', -NR'R", -SR', -halogen, -SiR'R"R"', -OC(O)R', - C(O)R', -CO₂R', -CONR'R", -OC(O)NR'R", -NR"C(O)R', -NR'-C(O)NR"R"', - NR"C(O)₂R', -NR-C(NR'R"R"')=NR"", -NR-C(NR'R")=NR"', -S(O)R', -S(O)₂R', - S(O)₂NR'R", -NRSO₂R', -CN and -NO₂ in a number ranging from zero to (2m'+1), where m' is the total number of carbon atoms in such radical. R', R", R'" and R"" each preferably independently refer to hydrogen, substituted or unsubstituted heteroalkyl, substituted or unsubstituted aryl, *e.g.*, aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R"' and R"" groups when more than one of these groups is present. When R' and R" are attached to the same nitrogen atom, they can be combined with the nitrogen atom to form a 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (*e.g.*, -CF₃ and -CH₂CF₃) and acyl (*e.g.*, -C(O)CH₃, -C(O)CF₃, -C(O)CH₂OCH₃, and the like).

Similar to the substituents described for the alkyl radical, substituents for the aryl and heteroaryl groups are generically referred to as "aryl group substituents." The substituents are selected from, for example: halogen, -OR', =O, =NR', =N-OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR'", - S(O)R', -S(O)₂R', -S(O)₂NR'R", -NRSO₂R', -CN and NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R"' and R"" are preferably independently selected from hydrogen, substituted or unsubstituted alkyl, substituted or unsubstituted heteroalkyl, substituted or unsubstituted aryl and substituted or unsubstituted heteroaryl. When a compound of the invention includes more than one R group, for example, each of the R groups is independently selected as are each R', R", R'" and R"" groups when more than one of these groups is present. In the schemes that follow, the symbol X represents "R" as described above.

Two of the substituents on adjacent atoms of the aryl or heteroaryl ring may optionally be replaced with a substituent of the formula -T-C(O)-(CRR')_{q}-U-, wherein T and U are independently NR-, -O-, -CRR'- or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula - (CRR')ₛ-X-(CR"R"')_{d}-, where s and d are independently integers of from 0 to 3, and X is -O-, -NR'-, -S-, -S(O)-, -S(O)₂-, or -S(O)₂NR'-. The substituents R, R', R" and R'" are preferably independently selected from hydrogen or substituted or unsubstituted (C₁-C₆)alkyl.

As used herein, the term "heteroatom" is meant to include oxygen (O), nitrogen (N), sulfur (S) and silicon (Si).

The use of reactive derivatives of PEG (or other linkers) to attach one or more peptide moieties to the linker is within the scope of the present invention. The invention is not limited by the identity of the reactive PEG analogue. Many activated derivatives of poly(ethyleneglycol) are available commercially and in the literature. It is well within the abilities of one of skill to choose, and synthesize if necessary, an appropriate activated PEG derivative with which to prepare a substrate useful in the present invention. *See,* Abuchowski et al. Cancer Biochem. Biophys., 7: 175-186 (1984); Abuchowski et al., J. Biol. Chem., 252: 3582-3586 (1977); Jackson et al., Anal. Biochem., 165: 114-127 (1987); Koide et al., Biochem Biophys. Res. Commun., 111: 659-667 (1983)), tresylate (Nilsson et al., Methods Enzymol., 104: 56-69 (1984); Delgado et al., Biotechnol. Appl. Biochem., 12: 119-128 (1990)); N-hydroxysuccinimide derived active esters (Buckmann et al., Makromol. Chem., 182: 1379-1384 (1981); Joppich et al., Makromol. Chem., 180: 1381-1384 (1979); Abuchowski et al., Cancer Biochem. Biophys., 7: 175-186 (1984); Katreet al. Proc. Natl. Acad. Sci. U.S.A., 84: 1487-1491 (1987); Kitamura et al., Cancer Res., 51: 4310-4315 (1991); Boccu et al., Z. Naturforsch., 38C: 94-99 (1983), carbonates (Zalipsky et al., POLY(ETHYLENE GLYCOL) CHEMISTRY: BIOTECHNICAL AND BIOMEDICAL APPLICATIONS, Harris, Ed., Plenum Press, New York, 1992, pp. 347-370; Zalipsky et al., Biotechnol. Appl. Biochem., 15: 100-114 (1992); Veronese et al., Appl. Biochem. Biotech., 11: 141-152 (1985)), imidazolyl formates (Beauchamp et al., Anal. Biochem., 131: 25-33 (1983); Berger et al., Blood, 71: 1641-1647 (1988)), 4-dithiopyridines (Woghiren et al., Bioconjugate Chem., 4: 314-318 (1993)), isocyanates (Byun et al., ASAIO Journal, M649-M-653 (1992)) and epoxides (U.S. Pat. No. 4,806,595, issued to Noishiki et al., (1989). Other linking groups include the urethane linkage between amino groups and activated PEG. *See,* Veronese, et al., Appl. Biochem. Biotechnol., 11: 141-152 (1985).

The term "amino acid" refers to naturally occurring and synthetic amino acids, as well as amino acid analogs and amino acid mimetics. Naturally occurring amino acids are those encoded by the genetic code, as well as those amino acids that are later modified, *e.g.*, hydroxyproline, γ-carboxyglutamate, and O-phosphoserine. Amino acid analogs refers to compounds that have the same basic chemical structure as a naturally occurring amino acid, *i.e.*, an α carbon that is bound to a hydrogen, a carboxyl group, an amino group, and an R group, *e.g.*, homoserine, norleucine, methionine sulfoxide, methionine methyl sulfonium. Such analogs have modified R groups (*e.g.*, norleucine) or modified peptide backbones, but retain the same basic chemical structure as a naturally occurring amino acid. "Amino acid mimetics" refers to chemical compounds having a structure that is different from the general chemical structure of an amino acid, but that functions in a manner similar to a naturally occurring amino acid.

"Peptide" refers to a polymer in which the monomers are amino acids, amino acid analogues and/or amino acid mimetics and are joined together through amide bonds, alternatively referred to as a polypeptide. Additionally, unnatural amino acids, for example, β-alanine, phenylglycine and homoarginine are also included. Amino acids that are not gene-encoded may also be used in the present invention. Furthermore, amino acids that have been modified to include reactive groups, glycosylation sites, polymers, therapeutic moieties, biomolecules and the like may also be used in the invention. All of the amino acids used in the present invention may be either the D - or L -isomer. The L -isomer is generally preferred. In addition, other peptidomimetics are also useful in the present invention. As used herein, "peptide" refers to both glycosylated and unglycosylated peptides. Also included are petides that are incompletely glycosylated by a system that expresses the peptide. For a general review, *see*, Spatola, A. F., in CHEMISTRY AND BIOCHEMISTRY OF AMINO ACIDS, PEPTIDES AND PROTEINS, B. Weinstein, eds., Marcel Dekker, New York, p. 267 (1983).

The term "nucleoside" refers to a glycosylamine that is a component of a nucleic acid and that comprises a nitrogenous base linked either to β-D-ribofuranose to form a ribonucleoside, or to 2-deoxy-β-D-ribofuranose to form a deoyribonucleoside. The base may be a purine *e.g.*, adenine or guanosine, or a pyrimidine *e.g.*, thymidine, cytidine, uridine or pseudouridine. Nucleoside also includes the unusual nucleoside used by microorganisms.

The term "targeting moiety," as used herein, refers to species that will selectively localize in a particular tissue or region of the body. The localization is mediated by specific recognition of molecular determinants, molecular size of the targeting agent or conjugate, ionic interactions, hydrophobic interactions and the like. Other mechanisms of targeting an agent to a particular tissue or region are known to those of skill in the art. Exemplary targeting moieties include antibodies, antibody fragments, transferrin, HS-glycoprotein, coagulation factors, serum proteins, β-glycoprotein, G-CSF, GM-CSF, M-CSF, EPO and the like.

As used herein, "therapeutic moiety" means any agent useful for therapy including, but not limited to, antibiotics, anti-inflammatory agents, anti-tumor drugs, cytotoxins, and radioactive agents. "Therapeutic moiety" includes prodrugs ofbioactive agents, constructs in which more than one therapeutic moiety is bound to a carrier, e.g, multivalent agents. Therapeutic moiety also includes proteins and constructs that include proteins. Exemplary proteins include, but are not limited to, Erythropoietin (EPO), Granulocyte Colony Stimulating Factor (GCSF), Granulocyte Macrophage Colony Stimulating Factor (GMCSF), Interferon (e.g., Interferon-α, -β, -γ), Interleukin (e.g., Interleukin II), serum proteins (e.g., Factors VII, VIIa, VIII, IX, and X), Human Chorionic Gonadotropin (HCG), Follicle Stimulating Hormone (FSH) and Lutenizing Hormone (LH) and antibody fusion proteins (e.g. Tumor Necrosis Factor Receptor ((TNFR)/Fc domain fusion protein)).

As used herein, "anti-tumor drug" means any agent useful to combat cancer including, but not limited to, cytotoxins and agents such as antimetabolites, alkylating agents, anthracyclines, antibiotics, antimitotic agents, procarbazine, hydroxyurea, asparaginase, corticosteroids, interferons and radioactive agents. Also encompassed within the scope of the term "anti-tumor drug," are conjugates of peptides with anti-tumor activity, *e.g.* TNF-α. Conjugates include, but are not limited to those formed between a therapeutic protein and a glycoprotein of the invention. A representative conjugate is that formed between PSGL-1 and TNF-α.

As used herein, "a cytotoxin or cytotoxic agent" means any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracinedione, mitoxantrone, mithramycin, actinomycin D, 1-dehydrotestosterone, glucocorticoids, procaine, tetracaine, lidocaine, propranolol, and puromycin and analogs or homologs thereof. Other toxins include, for example, ricin, CC-1065 and analogues, the duocarmycins. Still other toxins include diptheria toxin, and snake venom (*e.g.*, cobra venom).

As used herein, "a radioactive agent" includes any radioisotope that is effective in diagnosing or destroying a tumor. Examples include, but are not limited to, indium-111, cobalt-60. Additionally, naturally occurring radioactive elements such as uranium, radium, and thorium, which typically represent mixtures of radioisotopes, are suitable examples of a radioactive agent. The metal ions are typically chelated with an organic chelating moiety.

Many useful chelating groups, crown ethers, cryptands and the like are known in the art and can be incorporated into the compounds of the invention (*e.g.*, EDTA, DTPA, DOTA, NTA, HDTA, *etc.* and their phosphonate analogs such as DTPP, EDTP, HDTP, NTP, *etc*). *See,* for example, Pitt et al., "The Design of Chelating Agents for the Treatment of Iron Overload," In, INORGANIC CHEMISTRY IN BIOLOGY AND MEDICINE; Martell, Ed.; American Chemical Society, Washington, D.C., 1980, pp. 279-312; Lindoy, THE CHEMISTRY OF MACROCYCLIC LIGAND COMPLEXES; Cambridge University Press, Cambridge, 1989; Dugas, BIOORGANIC CHEMISTRY; Springer-Verlag, New York, 1989, and references contained therein.

Additionally, a manifold of routes allowing the attachment of chelating agents, crown ethers and cyclodextrins to other molecules is available to those of skill in the art. *See,* for example, Meares et al., "Properties of In Vivo Chelate-Tagged Proteins and Polypeptides." In, MODIFICATION OF PROTEINS: FOOD, NUTRITIONAL, AND PHARMACOLOGICAL ASPECTS;" Feeney, et al., Eds., American Chemical Society, Washington, D.C.,1982, pp. 370-387; Kasina et al., Bioconjugate Chem., 9: 108-117 (1998); Song et al., Bioconjugate Chem., 8: 249-255 (1997).

### INTRODUCTION

The present invention provides for compounds is accordance with the appended claims.

The present invention provides polymeric species, and sugars, activated sugars, and nucleotide sugars that are conjugated to these polymers. The polymeric conjugates of the nucleotide sugars are generally substrates for an enzyme that transfers the sugar moiety and its polymeric substituent onto an appropriate acceptor moiety of a substrate. Accordingly, the invention also provides substrates modified by glycoconjugation using a polymeric conjugate of a nucleotide sugar and an appropriate enzyme. Substrates that can be glycoconjugated using a compound of the invention include peptides, e.g., glycopeptides, lipids, e.g., glycolipids and aglycones (sphingosines, ceramides).

As discussed in the preceding sections, art-recognized chemical methods of covalent PEGylation rely on chemical conjugation through reactive groups on amino acids or carbohydrates. Through careful design of the conjugate and the reaction conditions, useful conjugates have been prepared using chemically-mediated conjugation strategies. A major shortcoming of chemical conjugation of polymers to proteins or glycoproteins is the lack of selectivity of the activated polymers, which often results in attachment of polymers at sites implicated in protein or glycoprotein bioactivity. Several strategies have been developed to address site selective conjugation chemistries, however, only one universal method suitable for a variety of recombinant proteins has been developed.

In contrast to art-recognized methods, the present invention provides compounds that are of use in a novel strategy for highly selective, site-directed glycoconjugation of branched water-soluble polymers, *e.g.*, glyco-PEGylation. In an exemplary embodiment of the invention, site directed attachment of branched water-soluble polymers is accomplished by *in vitro* enzymatic glycosylation of specific peptide sequences using a nucleotide sugar or activated sugar of the invention. Glyco-conjugation can be performed enzymatically utilizing a glycosyltransferase, *e.g.*, a sialyltransferase, capable of transferring the species branched water-soluble polymer-sugar, *e.g.*, PEG-sialic acid, to a glycosylation site ("glyco-PEGylation").

As discussed above, the present invention provides a conjugate between a sugar having any desired carbohydrate structure, modified with a polymeric moiety. Sugar nucleotides and activated sugars based on these sugar structures are also a component of the invention. The polymeric modifying moiety is attached to the sugar moiety by enzymatic means, chemical means or a combination thereof, thereby producing a modified nucleotide sugar. The sugars are substituted with the polymeric modifying moiety at any desired position. In an exemplary embodiment, the sugar is a furanose that is substituted at one or more of C-1, C-2, C-3, C-4 or C-5. In another embodiment, the invention provides a pyranose that is substituted with the polymeric modifying moiety at one or more of C-1, C-2, C-3, C-4, C-5 or C-6. Preferably, the polymeric modifying moiety is attached directly to an oxygen, nitrogen or sulfur pendent from the carbon. Alternatively, the polymeric modifying moiety is attached to a linker that is interposed between the sugar and the modifying moiety. The linker is attached to an oxygen, nitrogen or sulfur pendent from the selected carbon.

In a presently preferred embodiment, the polymeric modifying moiety is appended to a position, that is selected such that the resulting conjugate functions as a substrate for an enzyme used to ligate the modified sugar moiety to another species, e.g., peptide, glycopeptide, lipid, glycolipid, etc. Exemplary enzymes are discussed in greater detail herein and include glycosyl transferases (sialyl transferases, glucosyl transferases, galactosyl transferases, N-acetylglucosyl transferases, N-acetylgalactosyl transferases, mannosyl transferases, fucosyl transferases, etc.). Exemplary sugar nucleotide and activated sugar conjugates of the invention also include substrates for mutant glycosidases and mutant glycoceramidases that are modified to have synthetic, rather than hydrolytic activity.

In an exemplary embodiment, the conjugate of the invention includes a sugar, activated sugar or nucleotide sugar that is conjugated to one or more polymer, e.g. a branched polymer. Exemplary polymers include both water-soluble and water-insoluble species.

In an exemplary embodiment, the polymeric modifying group is directly or indirectly attached to a pyranose or a furanose. For example: 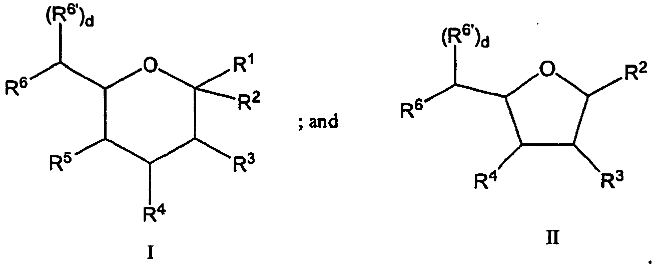 In Formulae I and II, R¹ is H, CH₂OR⁷, COOR⁷ or OR⁷, in which R⁷ represents H, substituted or unsubstituted alkyl or substituted or unsubstituted heteroalkyl. R² is H, OH, NH or a moiety that includes a nucleotide. An exemplary R² species according to this embodiment has the formula: 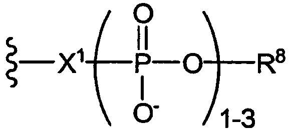 in which X¹ represents O or NH and R⁸ is a nucleoside.

The symbols R³, R⁴, R⁵, R⁶ and R⁶' independently represent H, substituted or unsubstituted alkyl, OR⁹, NHC(O)R¹⁰. The index d is 0 or 1. R⁹ and R¹⁰ are independently selected from H, substituted or unsubstituted alkyl, substituted or unsubstituted heteroalkyl or sialic acid. At least one of R³, R⁴, R⁵, R⁶, and R⁶' includes the polymeric modifying moiety, *e.g.*, PEG. In an exemplary embodiment, R⁶ and R^{6'}, together with the carbon to which they are attached are components of the side chain of sialic acid. In still a further exemplary embodiment, this side chain is modified with the polymeric modifying moiety (or a linker-polymeric modifying moiety) at one or more of C-6, C-7 or C-9.

The symbols R³, R⁴, R⁵ and R⁶ independently represent H, OR⁹, NHC(O)R¹⁰. R⁹ and R¹⁰ are independently selected from H, substituted or unsubstituted alkyl or substituted or unsubstituted heteroalkyl. At least one of R³, R⁴, R⁵, R⁶, or R^{6'} include the polymeric modifying moiety.

In another exemplary embodiment, the sugar moiety is a sialic acid moiety that has been oxidized and conjugated to a polymeric modifying moiety, such as is described in commonly assigned U.S. Provisional Patent Application No. (Attomey Docket No. 040853-01-5150), filed January 6, 2005.

Described herein are compounds wherein the polymeric modifying moiety is joined to the sugar core through a linker: 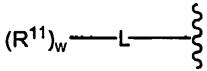 in which R¹¹ is the polymeric moiety and L is selected from a bond and a linking group, and w is an integer from 1-6, preferably 1-3 and more preferably, 1-2.

When L is a bond it is formed between a reactive functional group on a precursor of R¹¹ and a reactive functional group of complementary reactivity on a precursor of L. As set forth herein, the selection and preparation of precursors with appropriate reactive functional groups is within the ability of those skilled in the art. Moreover, combining the precursors proceed by chemistries that are well-understood in the art.

In an exemplary embodiment L is a linking group that is formed from an amino acid, an amino acid mimetic, providing a modified sugar in which the polymeric modifying moiety is attached through a substituted alkyl linker. The linker is formed through reaction of the amine moiety and carboxylic acid (or a reactive derivative, e.g., active ester, acid halide, etc.) of the amino acid with groups of complementary reactivity on the precursors to L and R¹¹. The elements of the conjugate can be conjugated in essentially any convenient order. For example the precursor to L can be in place on the saccharide core prior to conjugating the precursors of R¹¹ and L. Alternatively, an R¹¹-L cassette, bearing a reactive functionality on L can be prepared and subsequently linked to the saccharide through a reactive functional group of complementary reactivity on this species.

In an exemplary embodiments, the polymeric modifying moiety is R³ and/or R⁶. In another exemplary embodiment, R³ and/or R⁶ includes both the polymeric modifying moiety and a linker, L, joining the polymeric moiety to the remainder of the molecule. In another exemplary embodiment, the polymeric modifying moiety is R³. And, in a further exemplary embodiment, R³ includes both the polymeric modifying moiety and a linker, L, joining the polymeric moiety to the remainder of the molecule. In yet another exemplary embodiment in which the sugar is a sialic acid, the polymeric modifying moiety is at R⁵ or attached at a position of the sialic acid side chain, e.g., C-9.

### Branched Polymer Conjugates

In an exemplary embodiment, the polymeric modifying moiety is a branched structure that includes two or more polymeric chains attached to central moiety, having the formula: 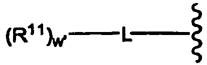 in which R¹¹ and L are as discussed above and w' is an integer from 2 to 6, preferably from 2 to 4 and more preferably from 2 to 3.

An exemplary precursor of use to form the conjugates according to this embodiment of the invention has the formula:

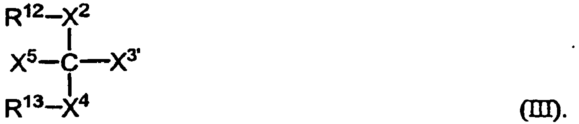

The branched polymer species according to this formula are essentially pure water-soluble polymers. X^{3'} is a moiety that includes an ionizable, e.g., COOH, H₂PO₄, HSO₃, HPO₃, etc.) or other reactive functional group, e.g., *infra.* C is carbon. X⁵ is preferably a non-reactive group (e.g., H, unsubstituted alkyl, unsubstituted heteroalkyl), and can be a polymeric arm. R¹² and R¹³ are independently selected polymeric arms, e.g., nonpeptidic, nonreactive polymeric arms. X² and X⁴ are linkage fragments that are preferably essentially non-reactive under physiological conditions, which may be the same or different. Alternatively, these linkages can include one or more moiety that is designed to degrade under physiologically relevant conditions, e.g., esters, disulfides, etc. X² and X⁴ join polymeric arms R¹² and R¹³ to C. When X^{3'} is reacted with a reactive functional group of complementary reactivity on a linker, sugar or linker-sugar cassette, X^{3'} is converted to a component of linkage fragment X³.

Exemplary linkage fragments for X² and X⁴ include S, SC(O)NH, HNC(O)S, SC(O)O, O, NH, NHC(O), (O)CNH and NHC(O)O, and OC(O)NH, CH₂S, CH₂O, CH₂CH₂O, CH₂CH₂S, (CH₂)ₐO, (CH₂)ₐS or (CH₂)ₐY'-PEG or (CH₂)ₐY'-PEG wherein Y' is S or O and a is an integer from 1 to 50.

In an exemplary embodiment, the precursor (III), or activated derivative thereof, is bound to the sugar, activated sugar or sugar nucleotide through a reaction between X^{3'} and a group of complementary reactivity on the sugar moiety. Alternatively, X^{3'} reacts with a reactive functional group on a precursor to linker, L. One or more of R¹, R³, R⁴, R⁵ or R⁶ of Formulae I and II can include the branched polymeric modifying moiety.

In an exemplary embodiment, the moiety: 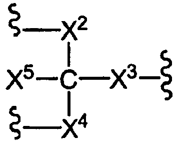 is the linker arm, L. In this embodiment, an exemplary linker is derived from a natural or unnatural amino acid, amino acid analogue or amino acid mimetic. The branched polymers found in the compounds of the invention have the formula:

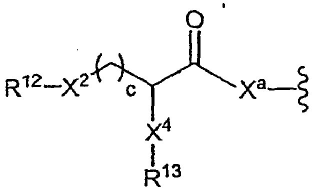

X^{a} is a linking moiety that is formed by the reaction of a reactive functional group on a precursor of the branched polymeric modifying moiety and the sugar moiety, or a precursor to a linker. For example, when X^{3'} is a carboxylic acid, it can be activated and bound directly to an amine group pendent from an amino-saccharide (e.g., GalNH₂, GlcNH₂, ManNH₂, etc.), forming an X^{a} that is an amide. Additional exemplary reactive functional groups and activated precursors are described hereinbelow. The index c represents an integer from 1 to 10. The other symbols have the same identity as those discussed above.

In another exemplary embodiment, X^{a} is a linking moiety formed with another linker: 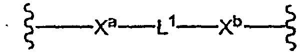 in which X^{b} is a linking moiety and is independently selected from those groups set forth for X^{a}, and L¹ is a bond, substituted or unsubstituted alkyl or substituted or unsubstituted heteroalkyl.

Exemplary species for X^{a} and X^{b} include S, SC(O)NH, HNC(O)S, SC(O)O, O, NH, NHC(O), (O)CNH and NHC(O)O, and OC(O)NH.

In the compounds of the invention, at least one of R³, R⁴, R⁵, R⁶ and R⁶ includes a moiety having the formula: 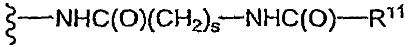 in which s is an integer from 0 to 20 and R¹¹ is a polymeric modifying moiety.

In a further exemplary embodiment, R⁶ includes the branched polymeric modifying group and the modified sugar or nucleotide sugar has a formula that is selected from: 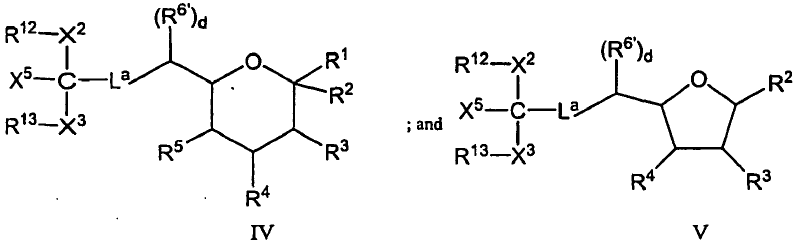 in which the identity of the radicals represented by the various symbols is the same as that discussed hereinabove. L^{a} is a substituted or unsubstituted alkyl or substituted or unsubstituted heteroalkyl moiety. In an exemplary embodiment, L^{a} is a moiety of the side chain of sialic acid that is functionalized with the polymeric modifying moiety as shown.

In yet another exemplary embodiment, the invention provides sugars and nucleotide sugars that have the formula: 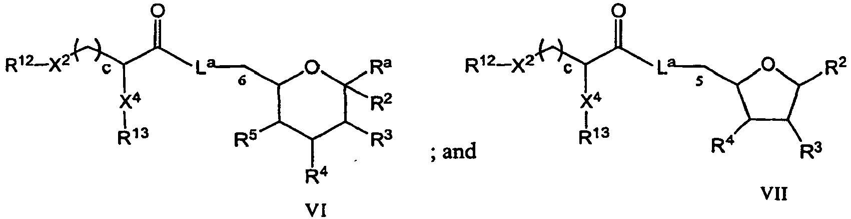 The identity of the radicals represented by the various symbols is the same as that discussed hereinabove. As those of skill will appreciate, the linker arm in Formulae VI and VIII is equally applicable to other modified sugars set forth herein.

The embodiments of the invention set forth above are further exemplified by reference to species in which the polymer is a water-soluble polymer, particularly poly(ethylene glycol) ("PEG"), e.g., methoxy-poly(ethylene glycol) ("m-PEG"). Those of skill will appreciate that the focus in the sections that follow is for clarity of illustration and the various motifs set forth using PEG as an exemplary polymer are equally applicable to species in which a polymer other than PEG is utilized.

### Water-Soluble Polymers

Many water-soluble polymers are known to those of skill in the art. The term water-soluble polymer encompasses species such as saccharides (*e.g*., dextran, amylose, hyalouronic acid, poly(sialic acid), heparans, heparins, etc.); poly (amino acids), *e.g.*, poly(aspartic acid) and poly(glutamic acid); nucleic acids; synthetic polymers (*e.g*., poly(acrylic acid), poly(ethers), *e.g*., poly(ethylene glycol); peptides, proteins, and the like. A polymer typically comprises at least two polymeric units. In an exemplary embodiment the polymer is from 2-25 units. In another exemplary embodiment the polymer comprises 2-8 polymeric units.

Methods for activation of polymers can also be found in WO 94/17039, U.S. Pat. No. 5,324,844, WO 94/18247, WO 94/04193, U.S. Pat. No. 5,219,564, U.S. Pat. No. 5,122,614, WO 90/13540, U.S. Pat. No. 5,281,698, and more WO 93/15189, and for conjugation between activated polymers and peptides, *e.g*. Coagulation Factor VIII (WO 94/15625), hemoglobin (WO 94/09027), oxygen carrying molecule (U.S. Pat. No. 4,412,989), ribonuclease and superoxide dismutase (Veronese at al., App. Biochem. Biotech. 11: 141-45 (1985)).

Preferred water-soluble polymers are those in which a substantial proportion of the polymer molecules in a sample of the polymer are of approximately the same molecular weight; such polymers are "homodisperse."

The present invention is further illustrated by reference to a poly(ethylene glycol) conjugate. Several reviews and monographs on the functionalization and conjugation of PEG are available. *See,* for example, Harris, Macronol. Chem. Phys. C25: 325-373 (1985); Scouten, Methods in Enzymology 135: 30-65 (1987); Wong et al., Enzyme Microb. Technol. 14: 866-874 (1992); Delgado et al., Critical Reviews in Therapeutic Drug Carrier Systems 9: 249-304 (1992); Zalipsky, Bioconjugate Chem. 6: 150-165 (1995); and Bhadra, et al., Pharmazie, 57:5-29 (2002). Routes for preparing reactive PEG molecules and forming conjugates using the reactive molecules are known in the art. For example, U.S. Patent No. 5,672,662 discloses a water soluble and isolatable conjugate of an active ester of a polymer acid selected from linear or branched poly(alkylene oxides), poly(oxyethylated polyols), poly(olefinic alcohols), and poly(acrylomorpholine).

U.S. Patent No. 6,376,604 sets forth a method for preparing a water-soluble 1-benzotriazolylcarbonate ester of a water-soluble and non-peptidic polymer by reacting a terminal hydroxyl of the polymer with di(1-benzotriazoyl)carbonate in an organic solvent. The active ester is used to form conjugates with a biologically active agent such as a protein or peptide.

WO 99/45964 describes a conjugate comprising a biologically active agent and an activated water soluble polymer comprising a polymer backbone having at least one terminus linked to the polymer backbone through a stable linkage, wherein at least one terminus comprises a branching moiety having proximal reactive groups linked to the branching moiety, in which the biologically active agent is linked to at least one of the proximal reactive groups. Other branched poly(ethylene glycols) are described in WO 96/21469, U.S. Patent No. 5,932,462 describes a conjugate formed with a branched PEG molecule that includes a branched terminus that includes reactive functional groups. The free reactive groups are available to react with a biologically active species, such as a protein or peptide, forming conjugates between the poly(ethylene glycol) and the biologically active species. U.S. Patent No. 5,446,090 describes a bifunctional PEG linker and its use in forming conjugates having a peptide at each of the PEG linker termini.

Conjugates that include degradable PEG linkages are described in WO 99/34833; and WO 99/14259, as well as in U.S. Patent No. 6,348,558.

The art-recognized methods of polymer activation set forth above are of use in the context of the present invention in the formation of the branched polymers set forth herein and also for the conjugation of these branched polymers to other species, e.g., sugars, sugar nucleotides and the like.

Exemplary modifying groups are discussed below. The modifying groups can be selected for their ability to impart to a peptide one or more desirable property. Exemplary properties include, but are not limited to, enhanced pharmacokinetics, enhanced pharmacodynamics, improved biodistribution, providing a polyvalent species, improved water solubility, enhanced or diminished lipophilicity, and tissue targeting.

Described poly(ethylene glycol) molecules include those having the formula: 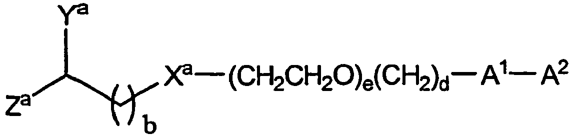 in which A² is H, OH, NH₂, substituted or unsubstituted alkyl, substituted or unsubstituted aryl, substituted or unsubstituted heteroaryl, substituted or unsubstituted heterocycloalkyl, substituted or unsubstituted heteroalkyl, *e.g*., acetal, OHC-, H₂N-(CH₂)_{q}-, HS-(CH₂)_{q}, or -(CH₂)_{q}C(Y^{b})Z^{b}. The index "e" represents an integer from 1 to 2500. The indices b, d, and q independently represent integers from 0 to 20. The symbols Z^{a} and Z^{b} independently represent OH, NH₂, leaving groups, *e.g*., imidazole, p-nitrophenyl, HOBT, tetrazole, halide, S-R^{a}, the alcohol portion of activated esters; -(CH²)ₚC(Y^{b})V, or -(CH₂)ₚU(CH₂)₅C(Y^{b})ᵥ. The symbol Y^{a} represents H(2), =O, =S, =N-R^{b}. The symbols X^{a}, Y^{a}, Y^{b}, A¹, and U independently represent the moieties O, S, N-R^{c}. The symbol V represents OH, NH₂, halogen, S-R^{a}, the alcohol component of activated esters, the amine component of activated amides, sugar-nucleotides, and proteins. The indices p, q, s and v are members independently selected from the integers from 0 to 20. The symbols R^{a}, R^{b}, and R^{c} independently represent H, substituted or unsubstituted alkyl, substituted or unsubstituted heteroalkyl, substituted or unsubstituted aryl, substituted or unsubstituted heterocycloalkyl and substituted or unsubstituted heteroaryl.

Specific embodiments of branched polymers, e.g., PEGs, of use in the invention include: 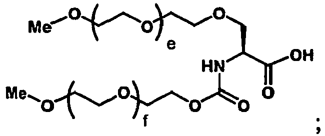 and carbonates and active esters of these species, such as: 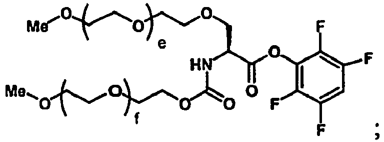 and 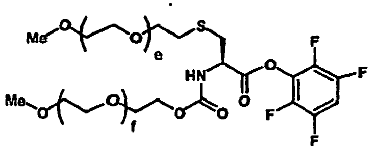 can be used to form the branched polymeric species, linker arm conjugates of these species and conjugates between these compounds and sugars and nucleotide sugars. The indices e and f are independently selected from 1 to 2500.

Other exemplary activating, or leaving groups, appropriate for activating linear PEGs of use in preparing the compounds set forth herein include, but are not limited to the species: 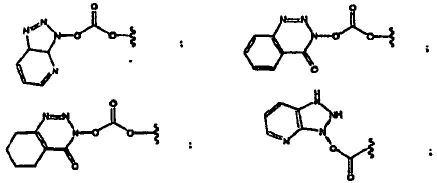 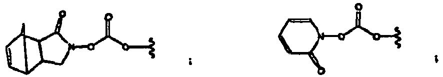 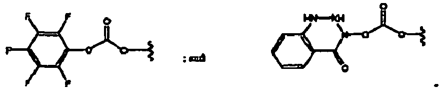 It is well within the abilities of those of skill in the art to select an appropriate activating group for a selected moiety on the precursor to the polymeric modifying moiety.

PEG molecules that are activated with these and other species and methods of making the activated PEGs are set forth in WO 04/083259.

In exemplary embodiments, the branched polymer is a PEG based upon a cysteine, serine, lysine, di- or tri-lysine core. Thus, branched PEGs include: 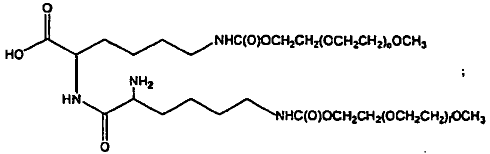 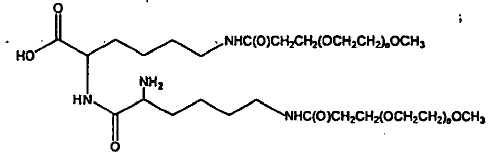 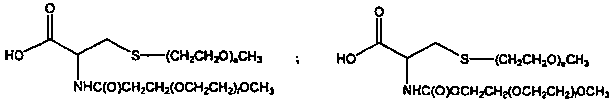 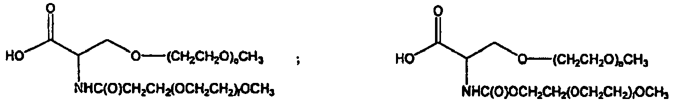 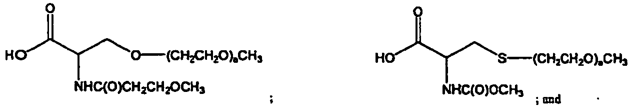 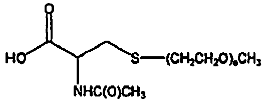 The indices e and f are independently selected from 1 to 2500.

Also described are branched PEG moieties based upon a tri-lysine peptide. The tri-lysine can be mono-, di-, tri-, or tetra-PEG-ylated. Exemplary species according to this embodiment have the formulae: 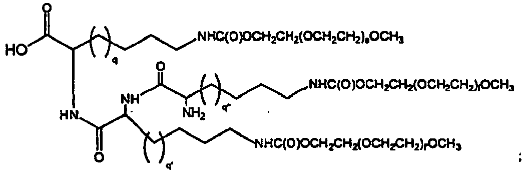 and 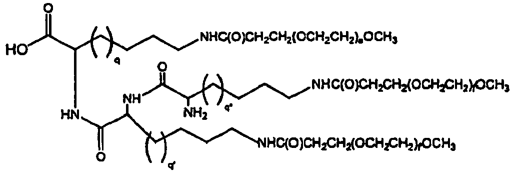 in which e, f and f are independently selected integers from 1 to 2500; and q, q' and q" are independently selected integers from 0 to 20.

An exemplary branched PEG species is a lysine, serine- or cysteine-(m-PEG)₂ in which the m-PEG is a 20 kD m-PEG.

Those of skill in the art will appreciate that one or more of the m-PEG arms of the branched polymer can be replaced by a PEG moiety with a different terminus, e.g., OH, COOH, NH₂, C₂-C₁₀-alkyl, etc, although such compounds do not form part of the present invention. Moreover, the structures above are readily modified by inserting alkyl linkers (or removing carbon atoms) between the α-carbon atom and the functional group of the side chain. Thus, "homo" derivatives and higher homologues, as well as lower homologues are within the scope of cores for branched PEGs of use in the present invention.

The branched PEG species set forth herein are readily prepared by methods such as that set forth in the scheme below: 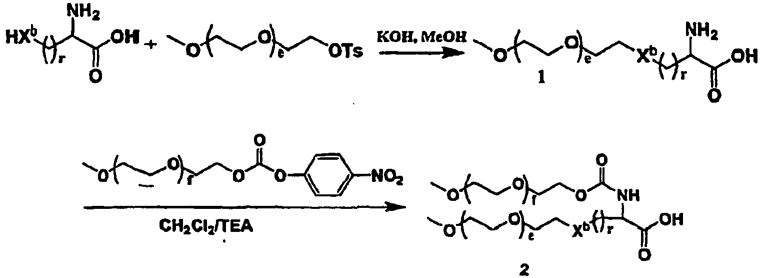 in which X^{b} is O, NH or S and r is an integer from 1 to 10. The indices e and f are independently selected integers from 1 to 2500. Exemplary branched PEG species are 10,000,15,000, 20,000, 30,000 and 40,000 daltons.

Thus, according to this scheme, a natural or unnatural amino acid is contacted with an activated m-PEG derivative, in this case the tosylate, forming **1** by alkylating the side-chain heteroatom X^{b}. The mono-functionalized m-PEG amino acid is submitted to N-acylation conditions with a reactive m-PEG derivative, thereby assembling branched m-PEG **2**. As one of skill will appreciate, the tosylate leaving group can be instead any suitable leaving group, *e.g*., halogen, mesylate, triflate, etc. Similarly, the reactive carbonate utilized to acylate the amine can be instead an active ester, *e.g*., N-hydroxysuccinimide, etc., or the acid can be activated *in situ* using a dehydrating agent such as dicyclohexylcarbodiimide, carbonyldiimidazole, etc.

In the exemplary scheme set forth above, the modifying group is a linear PEG moiety.

Also disclosed are compounds exemplified by branched cores functionalized with PEG, such as the examples set forth below: 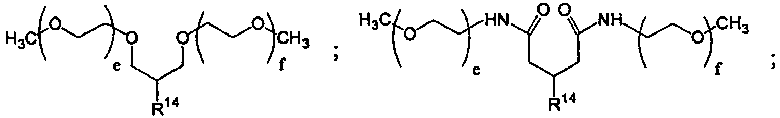 and 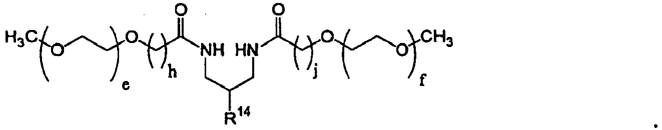 in which R¹⁴ is OH or another reactive functional group. An exemplary reactive functional group is C(O)Q', in which Q' is selected such that C(O)Q' is a reactive functional group. Exemplary species for Q' include halogen, NHS, pentafluorophenyl, HOBT, HOAt, and p-nitrophenyl. The index "e" and the index "f" are integers independently selected from 1 to 2500.

The branched compounds set forth above, and additional branched compounds are readily prepared from such starting materials as:

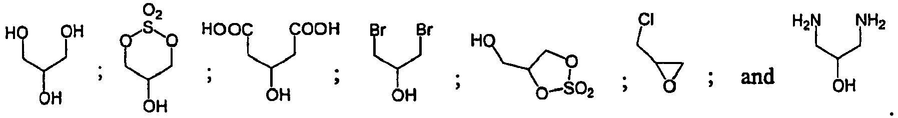

### Polymer Modified Sugar Species

The sugar moiety of the nucleotide sugars of the invention can be selected from both natural and unnatural furanoses and hexanoses. The unnatural saccharides optionally include an alkylated or acylated hydroxyl and/or amine moiety, e.g., ethers, esters and amide substituents on the ring. Other unnatural saccharides include an H, hydroxyl, ether, ester or amide substituent at a position on the ring at which such a substituent is not present in the natural saccharide. The sugar moiety can be a mono-, oligo- or poly-saccharide.

Exemplary natural sugars of use in the present invention include glucose, galactose, fucose, mannose, xylanose, ribose, N-acetyl glucose, sialic acid and N-acetyl galactose.

Similarly, the nucleoside can be selected from both natural and unnatural or unusual nucleosides. Exemplary natural nucleosides of use in the present invention include cytosine, thymine, guanine, adenine and uracil. Unusual nucleosides may include but are not limited to such molecules as spongouridin and spongothymidin. The art is replete with structures of unnatural and unusual nucleosides and methods of making them.

Exemplary modified sugar nucleotides of the invention include GDP-Man, GDP-Fuc, UDP-Gal, UDP-Gal-NH₂, UDP-GalNAc, UDP-Glc, UDP-Glc-NH₂, UDP-GlcNAc, UDP-Glc, UDP-GlcUA and CMP-Sia. As with the sugars of the invention discussed above, the sugar nucleotides of the invention can be substituted with a polymeric modifying moiety (or linker-modifying moiety) at any position of the saccharide. For example, compounds encompassed by the invention include those in which the L-R¹¹ moiety is conjugated to C-5 of a furanose-based nucleotide sugar or C-6 of a pyranose-based nucleotide sugar.

Exemplary moieties which could be attached to the conjugates disclosed herein include, but are not limited to, PEG derivatives (*e.g*., alkyl-PEG, acyl-PEG, acyl-alkyl-PEG, alkyl-acyl-PEG carbamoyl-PEG, aryl-PEG), PPG derivatives (*e.g*., alkyl-PPG, acyl-PPG, acyl-alkyl-PPG, alkyl-acyl-PPG carbamoyl-PPG, aryl-PPG), therapeutic moieties, diagnostic moieties, mannose-6-phosphate, heparin, heparan, SLeₓ, mannose, mannose-6-phosphate, Sialyl Lewis X, FGF, VFGF, proteins, chondroitin, keratan, dermatan, albumin, integrins, antennary oligosaccharides, peptides and the like. Methods of conjugating the various modifying groups to a saccharide moiety are readily accessible to those of skill in the art (POLY (ETHYLBNE GLYCOL CHEMISTRY : BIOTECHNICAL AND BIOMEDICAL APPLICATIONS, J. Milton Harris, Ed., Plenum Pub. Corp., 1992; POLY (ETHYLENE GLYCOL) CHEMICAL AND BIOLOGICAL APPLICATIONS, J. Milton Harris, Ed., ACS Symposium Series No. 680, American Chemical Society, 1997; Hermanson, BIOCONJUGATE TECHNIQUES, Academic Press, San Diego, 1996; and Dunn et al., Eds. POLYMERIC DRUGS AND DRUG DELIVERY SYSTEMS, ACS Symposium Series Vol. 469, American Chemical Society, Washington, D.C. 1991).

Exemplary sugar nucleotides of the present invention, in their modified form, include nucleotide mono-, di- or triphosphates or analogs thereof of a UDP-glycoside, CMP-glycoside, or a GDP-glycoside. Even more preferably, the modified sugar nucleotide is selected from an UDP-galactose, UDP-galactosamine, UDP-glucose, UDP-glucosamine, GDP-mannose, GDP-fucose, CMP-sialic acid, or CMP-NeuAc. N-acetylamine derivatives of the sugar nucleotides are also of use in the method of the invention.

In other embodiments, the modified sugar is an activated sugar. Activated modified sugars, which are useful in the present invention are typically glycosides which have been synthetically altered to include an activated leaving group. As used herein, the term "activated leaving group" refers to those moieties, which are easily displaced in enzyme-regulated nucleophilic substitution reactions. Many activated sugars are known in the art. See, for example, Vocadlo et al., In CARBOHYDRATE CHEMISTRY AND BIOLOGY, Vol. 2, Ernst et al. Ed., Wiley-VCH Verlag: Weinheim, Germany, 2000; Kodama et al., Tetrahedron Lett. 34: 6419 (1993); Lougheed, et al., .J. Biol. Chem. 274: 37717 (1999)).

Examples of activating groups (leaving groups) include fluoro, chloro, bromo, tosylate ester, mesylate ester, triflate ester and the like. Preferred activated leaving groups, for use in the present invention, are those that do not significantly sterically encumber the enzymatic transfer of the glycoside to the acceptor. Accordingly, preferred embodiments of activated glycoside derivatives include glycosyl fluorides and glycosyl mesylates, with glycosyl fluorides being particularly preferred. Among the glycosyl fluorides, α-galactosyl fluoride, α-mannosyl fluoride, α-glucosyl fluoride, α-fucosyl fluoride, α-xylosyl fluoride, α-sialyl fluoride, α-N-acetylglucosaminyl fluoride, α-N-acetylgalactosaminyl fluoride, β-galactosyl fluoride, β-mannosyl fluoride, β-glucosyl fluoride, β-fucosyl fluoride, β-xylosyl fluoride, β-sialyl fluoride, β-N-acetylglucosaminyl fluoride and β-N-acetylgalactosaminyl fluoride are most preferred.

By way of illustration, glycosyl fluorides can be prepared from the free sugar by first acetylating the sugar and then treating it with HF/pyridine. This generates the thermodynamically most stable anomer of the protected (acetylated) glycosyl fluoride (*i.e*., the α-glycosyl fluoride). If the less stable anomer (*i.e*., the β-glycosyl fluoride) is desired, it can be prepared by converting the peracetylated sugar with HBr/HOAc or with HCI to generate the anomeric bromide or chloride. This intermediate is reacted with a fluoride salt such as silver fluoride to generate the glycosyl fluoride. Acetylated glycosyl fluorides may be deprotected by reaction with mild (catalytic) base in methanol (*e.g*. NaOMe/MeOH). In addition, many glycosyl fluorides are commercially available.

Other activated glycosyl derivatives can be prepared using conventional methods known to those of skill in the art. For example, glycosyl mesylates can be prepared by treatment of the fully benzylated hemiacetal form of the sugar with mesyl chloride, followed by catalytic hydrogenation to remove the benzyl groups.

In a further exemplary embodiment, the modified sugar is an oligosaccharide having an antennary structure. In another embodiment, one or more of the termini of the antennae bear the modifying moiety. When more than one modifying moiety is attached to an oligosaccharide having an antennary structure, the oligosaccharide is useful to "amplify" the modifying moiety; each oligosaccharide unit conjugated to the peptide attaches multiple copies of the modifying group to the peptide. The general structure of a typical conjugate of the invention as set forth in the drawing above, encompasses multivalent species resulting from preparing a conjugate of the invention utilizing an antennary structure. Many antennary saccharide structures are known in the art, and the present method can be practiced with them without limitation.

In an exemplary embodiment, the activated, modified sugar is a substrate for a mutant enzyme that transfers the sugar onto an appropriate acceptor moiety of a substrate. Exemplary mutant enzymes include, e.g., those set forth in commonly assigned PCT publications WO03/046150 and WO03/045980

Water-soluble polymer modified sugar, activated sugar and nucleotide sugar species in which the sugar moiety is modified with a water-soluble polymer, e.g., a water-soluble polymer, are of use in the present invention. An exemplary modified sugar nucleotide bears a sugar group that is modified through an amine moiety on the sugar. Modified sugar nucleotides, e.g., saccharyl-amine derivatives of a sugar nucleotide, are also of use in the methods of the invention. For example, a saccharyl amine (without the modifying group) can be enzymatically conjugated to a peptide (or other species) and the free saccharyl amine moiety subsequently conjugated to a desired modifying group. Alternatively, the modified sugar nucleotide can function as a substrate for an enzyme that transfers the modified sugar to a saccharyl acceptor on a substrate, e.g., a peptide, glycopeptide, lipid, aglycone, glycolipid, etc.

In one embodiment, the sugar is conjugated to a branched polymeric species, such as those set forth herein.

In another embodiment, the sugar moiety is a modified sialic acid. When sialic acid is the sugar, the sialic acid is substituted with the modifying group at either the 9-position on the pyruvyl side chain or at the 5-position on the amine moiety that is normally acetylated in sialic acid.

In another embodiment, in which the saccharide core is galactose or glucose, R⁵ is NHC(O)Y.

In an exemplary embodiment, the modified sugar is based upon a 6-amino-N-acetyl-glycosyl moiety. As shown below for N-acetylgalactosamine, the 6-amino-sugar moiety is readily prepared by standard methods: 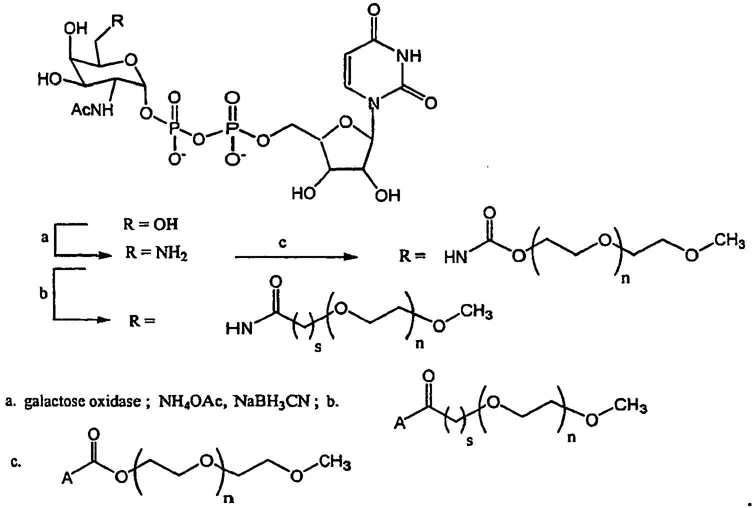 In the scheme above, the index n represents an integer from 1 to 2500, preferably from 10 to 1500, and more preferably from 10 to 1200. The symbol "A" represents an activating group, *e.g*., a halo, a component of an activated ester (*e.g*., a N-hydroxysuccinimide ester), a component of a carbonate (*e.g*., p-nitrophenyl carbonate) and the like. Those of skill in the art will appreciate that other PEG-amide nucleotide sugars are readily prepared by this and analogous methods. For the preparation of the compounds of the invention; a branched polymer, as set forth herein, can be substituted for the linear PEG.

An exemplary polymerically modified nucleotide sugar of the invention in which the C-6 position is modified has the formula: 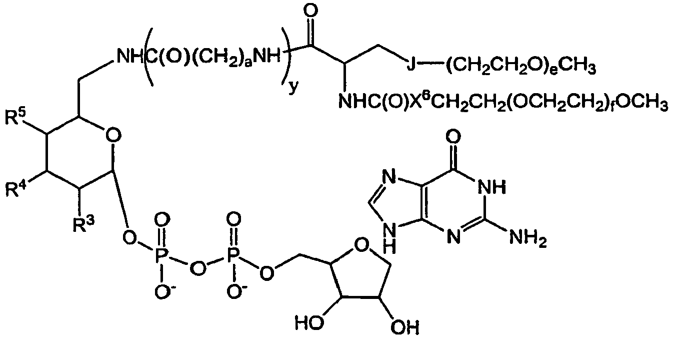 in which X⁶ is a bond or O, J is S or O, and y is 0 or 1. The indices e and f are independently selected from 1 to 2500.

In other exemplary embodiments, the amide moiety is replaced by a group such as a urethane or a urea.

In the discussion that follows, a number of specific examples of modified sugars that are useful in practicing the present invention are set forth. In the exemplary embodiments, a sialic acid derivative is utilized as the sugar nucleus to which the modifying group is attached. The focus of the discussion on sialic acid derivatives is for clarity of illustration only and should not be construed to limit the scope of the invention. Those of skill in the art will appreciate that a variety of other sugar moieties can be activated and derivatized in a manner analogous to that set forth using sialic acid as an example. For example, numerous methods are available for modifying galactose, glucose, N-acetylgalactosamine and fucose to name a few sugar substrates, which are readily modified by art recognized methods. *See,* for example, Elhalabi et al., Curr. Med. Chem. 6: 93 (1999); and Schafer et al., J. Org. Chem. 65: 24 (2000)).

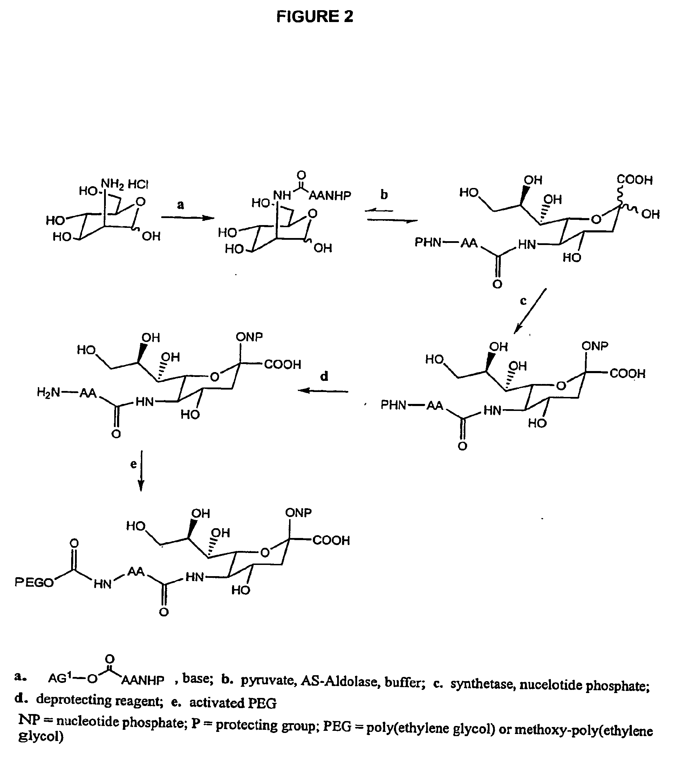

In **FIG. 2**, a general scheme according to the present invention is set forth. Thus, according to **FIG. 2**, an amide conjugate between mannosamine and a protected amino acid is formed by contacting mannosamine with an N-protected amino acid under conditions appropriate to form the conjugate. The carboxyl terminus of the protected amino acid is activated *in situ* or it is optionally converted to a reactive group that is stable to storage, e.g., N-hydroxy-succinimide. The amino acid can be selected from any natural or non-natural amino acid. Those of skill in the art understand how to protect side-chain amino acids from undesirably reacting in the method of the invention. The amide conjugate is reacted with pyruvate and sialic acid aldolase under conditions appropriate to convert the amide conjugate to a sialic acid amide conjugate, which is subsequently converted to a nucleotide phosphate sialic acid amide conjugate by reaction of the sialic acid amide conjugate with a precursor of the nucleotide phosphate and an appropriate enzyme. In an exemplary embodiment, the precursor is cytidine triphosphate and the enzyme is a synthetase. Following the formation of the nucleotide sugar, the amino acid amine is deprotected, providing a free, reactive amine amine. The amine serves as a locus for conjugating the modifying moiety to the nucleotide sugar. In **FIG. 2**, the modifying moiety is exemplified by a water-soluble polymer, i.e., poly(ethylene glycol), e.g., PEG, m-PEG, etc.

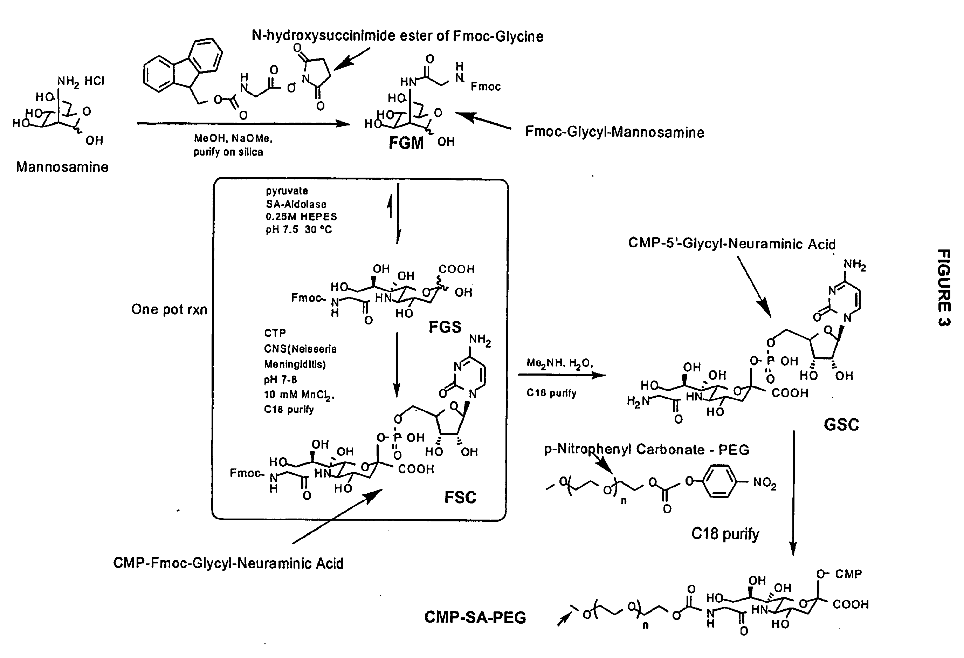

The present invention is further exemplified in **FIG. 3**, which sets forth a scheme for preparing sialic acid-glycyl-PEG-cytidine monophosphate. Similar to the scheme set forth in **FIG. 2**, that of **FIG. 3** originates with mannosamine. The sugar is conjugated with FMOC-glycine, using the N-hydroxysuccinimide activated derivative of the protected amino acid. The resulting amide conjugate is converted to the corresponding sialic acid by the action of sialic acid aldolase on the conjugate and pyruvate. The resulting sialic acid conjugate is converted to the cytidine monophosphate analogue using cytidine triphosphate and a synthetase. The CMP-analogue is deprotected by removing the protecting group from the amino acid amine moiety, converting this moiety to a reactive locus for conjugation. The amine moiety is reacted with an activated PEG species (m-PEG-O-nitrophenyl carbonate), thereby forming the sialic acid-glycyl-PEG-cytidine monophosphate.

Exemplary sugar cores based upon sialic acid have the formula: 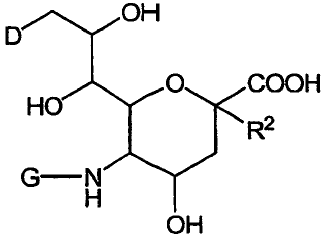 in which D is -OH or (R¹¹)_{w'}-L-. The symbol G represents H, (R¹¹)_{w'}-L- or -C(O)(C₁-C₆)alkyl. R¹¹ is as is as described above. At least one of D and G is R¹¹-L-.

Selected sialic acid-based nucleotide sugars functionalized with a branched polymer have the formula: 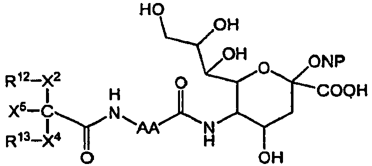 in which AA is an amino acid residue, PEG is poly(ethylene glycol) or methoxy-poly(ethylene glycol) and NP is a nucleotide, which is linked to the glycosyl moiety via a phosphodiester bond ("nucleotide phosphate"). Those of skill will appreciate that ONP can be replaced by an activating moiety as discussed herein.

Also described are sialic acid derivatives having a structure that is a member selected from: 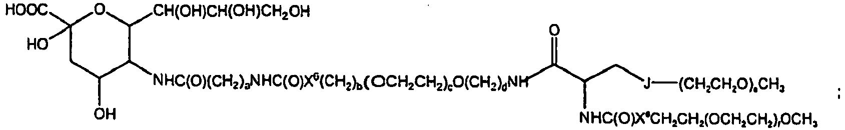 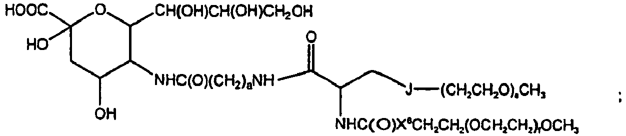 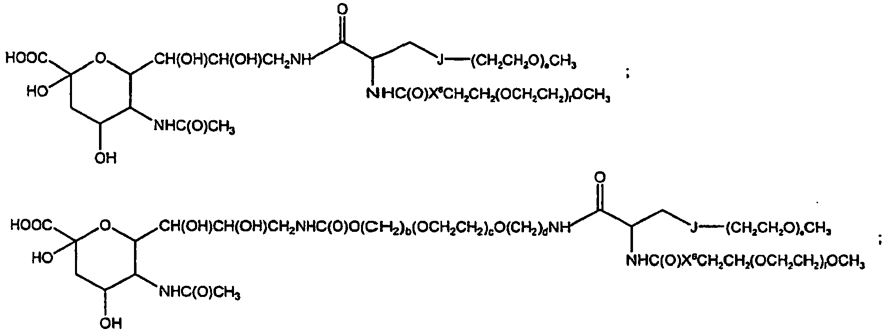 and 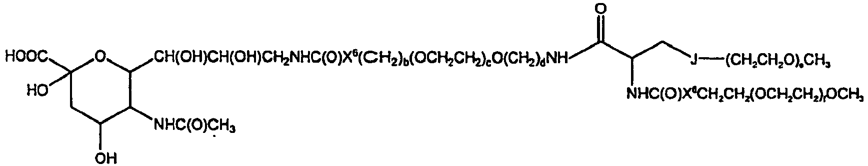 in which X⁶ is a bond or O, and J is S or O. The indices a, b and c are independently selected from 0 to 20, and e and f are independently selected from 1 to 2500. The second of these is a compound of the invention

Moreover, as discussed above, the present invention provides nucleotide sugars that are modified with a water-soluble polymer, which is branched. Also described are nucleotide sugars : 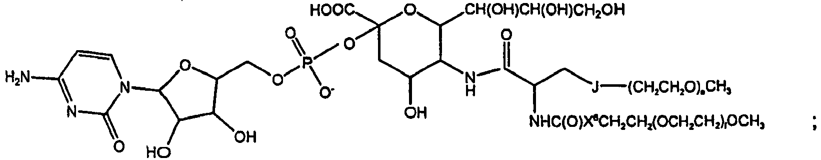 and 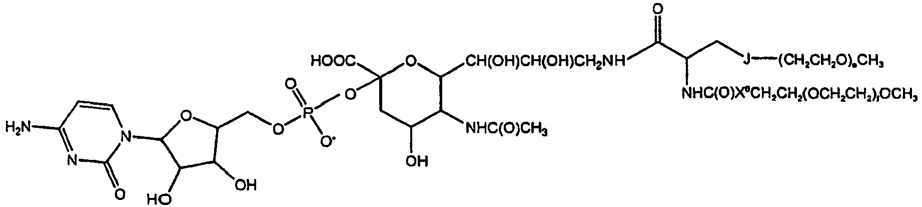 in which X⁶ is O or a bond, and J is S or O. The indices e and f are independently selected from 1 to 2500.

Also provided are conjugates of peptides and glycopeptides, lipids and glycolipids that include the compositions of the invention. The conjugates are formed by combining a nucleotide sugar or activated sugar of the invention and a substrate with an appropriate acceptor moiety for the sugar moiety and an enzyme for which the modified nucleotide sugar is a substrate under conditions appropriate to transfer the modified sugar from the nucleotide sugar onto the acceptor moiety. For example, herein described are conjugates having the following formulae: 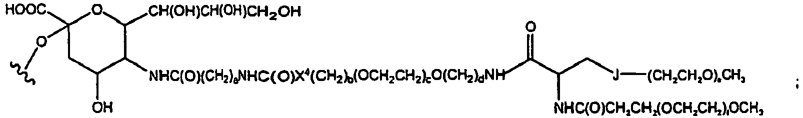 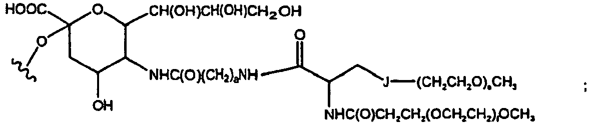 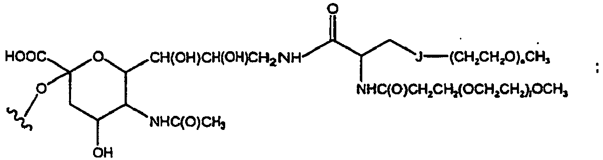 and 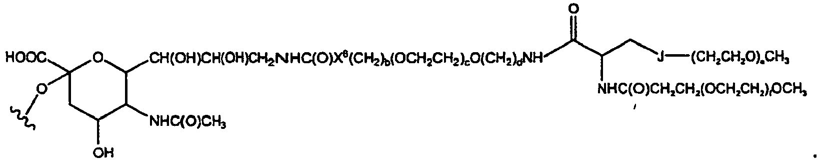 wherein J and X⁶ are as discussed above. The indices a, b, c, e and f are as discussed above.

Selected compounds of the invention are based on species having the stereochemistry of mannose, galactose and glucose. The general formulae of these compounds are: 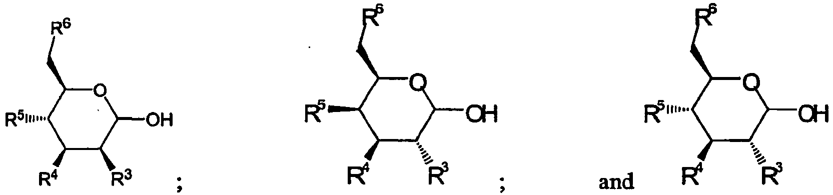 in which one of R³-R⁶ is the modifying moiety, e.g., polymeric modifying moiety or the polymeric modifying moiety-linker construct.

As discussed above, certain compounds of the present invention are polymeric modified sugar nucleotides. Exemplary sugar nucleotides that are used in the present invention in their modified form include nucleotide mono-, di- or triphosphates or analogs thereof. In a preferred embodiment, the modified sugar nucleotide is selected from a UDP-glycoside, CMP-glycoside, or a GDP-glycoside. Even more preferably, the modified sugar nucleotide is selected from an UDP-galactose, UDP-galactosamine, UDP-glucose, UDP-glucosamine, GDP-mannose, GDP-fucose, CMP-sialic acid, or CMP-Sia. In an exemplary embodiment, the nucleotide mono- di- or tri-phosphate is attached to C-1.

The saccharyl-amine derivatives of the sugar nucleotides are also of use in the method of the invention. For example, the saccharyl amine (without the modifying group) can be enzymatically conjugated to a peptide (or other species) and the free saccharyl amine moiety subsequently conjugated to a desired modifying group.

The sugar nucleotide conjugates of the invention are described generically by the formula: 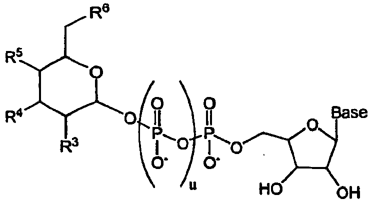 in which the symbols represent groups as discussed above. When the sugar core is mannose, the polymeric modifying moiety is preferably at R³, R⁴ or R⁶. For glucose, the polymeric modifying moiety is optionally at R⁵ or R⁶. The index "u" is 0, 1 or 2.

A linear PEG-modified nucleotide sugar based on GDP mannose has the structure:

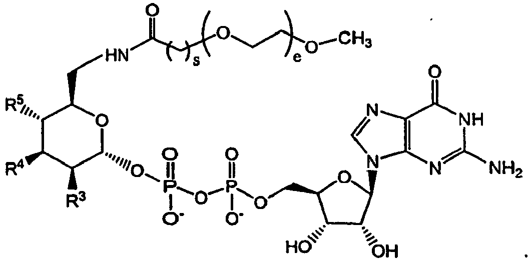

A linear PEG-nucleotide sugar conjugate, based on UDP galactose having the structure:

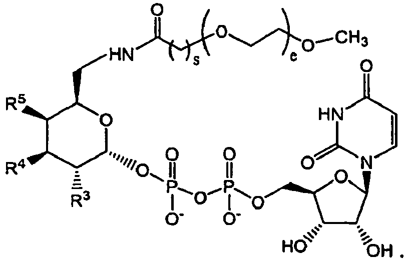

Another linear PEG-modified nucleotide sugar is based on glucose and has the formula: 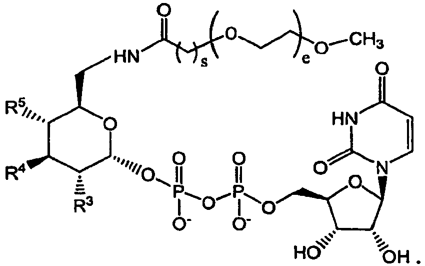 In each of the three preceding formulae, the identity of the radicals and indices is as discussed above.

To form compounds of the invention the linear PEG moiety can be replaced by a branched polymeric species as described herein.

In one embodiment in which the saccharide core is galactose or glucose, R⁵ is NHC(O)Y.

### Water-insoluble polymers

In another embodiment (not forming part of the invention, but discussed herein), analogous to those discussed above, the modified sugars include a water-insoluble polymer, rather than a water-soluble polymer. A water-insoluble polymer, like a water soluble polymer is typically comprised of at least two polymeric units. In one exemplary embodiment the polymer is comprised of from 2 to 25 polymeric units. In another exemplary embodiment the polymer is comprised of 2 to 8 polymeric units. The conjugates of the invention may also include one or more water-insoluble polymers. This embodiment of the invention is illustrated by the use of the conjugate as a vehicle with which to deliver a therapeutic peptide in a controlled manner. Polymeric drug delivery systems are known in the art. *See,* for example, Dunn et al., Eds. POLYMERIC DRUGS AND DRUG DELIVERY SYSTEMS, ACS Symposium Series Vol. 469, American Chemical Society, Washington, D.C. 1991. Those of skill in the art will appreciate that substantially any known drug delivery system is applicable to the described conjugates.

Representative water-insoluble polymers include, polyphosphazines, poly(vinyl alcohols), polyamides, polycarbonates, polyalkylenes, polyacrylamides, polyalkylene glycols, polyalkylene oxides, polyalkylene terephthalates, polyvinyl ethers, polyvinyl esters, polyvinyl halides, polyvinylpyrrolidone, polyglycolides, polysiloxanes, polyurethanes, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate) polyethylene, polypropylene, poly(ethylene glycol), poly(ethylene oxide), poly (ethylene terephthalate), poly(vinyl acetate), polyvinyl chloride, polystyrene, polyvinyl pyrrolidone, pluronics and polyvinylphenol and copolymers thereof.

Synthetically modified natural polymers of use in the described conjugates include, alkyl celluloses, hydroxyalkyl celluloses, cellulose ethers, cellulose esters, and nitrocelluloses. Particularly preferred members of the broad classes of synthetically modified natural polymers include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulfate sodium salt, and polymers of acrylic and methacrylic esters and alginic acid.

These and the other polymers discussed herein can be readily obtained from commercial sources such as Sigma Chemical Co. (St. Louis, MO.), Polysciences (Warrenton, PA.), Aldrich (Milwaukee, WI.), Fluka (Ronkonkoma, NY), and BioRad (Richmond, CA), or else synthesized from monomers obtained from these suppliers using standard techniques.

Representative biodegradable polymers of use in the described conjugates include polylactides, polyglycolides and copolymers thereof, poly(ethylene terephthalate), poly(butyric acid), poly(valeric acid), poly(lactide-co-caprolactone), poly(lactide-co-glycolide), polyanhydrides, polyorthoesters, blends and copolymers thereof. Of particular use are compositions that form gels, such as those including collagen, pluronics and the like.

The polymers described include "hybrid' polymers that include water-insoluble materials having within at least a portion of their structure, a bioresorbable molecule. An example of such a polymer is one that includes a water-insoluble copolymer, which has a bioresorbable region, a hydrophilic region and a plurality of crosslinkable functional groups per polymer chain.

For purposes of the present description, "water-insoluble materials" includes materials that are substantially insoluble in water or water-containing environments. Thus, although certain regions or segments of the copolymer may be hydrophilic or even water-soluble, the polymer molecule, as a whole, does not to any substantial measure dissolve in water.

For purposes of the present decription, the term "bioresorbable molecule" includes a region that is capable of being metabolized or broken down and resorbed and/or eliminated through normal excretory routes by the body. Such metabolites or break down products are preferably substantially non-toxic to the body.

The bioresorbable region may be either hydrophobic or hydrophilic, so long as the copolymer composition as a whole is not rendered water-soluble. Thus, the bioresorbable region is selected based on the preference that the polymer, as a whole, remains water-insoluble. Accordingly, the relative properties, *i.e*., the kinds of functional groups contained by, and the relative proportions of the bioresorbable region, and the hydrophilic region are selected to ensure that useful bioresorbable compositions remain water-insoluble.

Exemplary resorbable polymers include, for example, synthetically produced resorbable block copolymers of poly(α-hydroxy-carboxylic acid)/poly(oxyalkylene, (*see*, Cohn et al., U.S. Patent No. 4,826,945). These copolymers are not crosslinked and are water-soluble so that the body can excrete the degraded block copolymer compositions. *See,* Younes et al., JBiomed. Mater. Res. 21: 1301-1316 (1987); and Cohn et al., J Biomed. Mater. Res. 22: 993-1009 (1988).

Presently preferred bioresorbable polymers include one or more components selected from poly(esters), poly(hydroxy acids), poly(lactones), poly(amides), poly(ester-amides), poly (amino acids), poly(anhydrides), poly(orthoesters), poly(carbonates), poly(phosphazines), poly(phosphoesters), poly(thioesters), polysaccharides and mixtures thereof. More preferably still, the bioresorbable polymer includes a poly(hydroxy) acid component. Of the poly(hydroxy) acids, polylactic acid, polyglycolic acid, polycaproic acid, polybutyric acid, polyvaleric acid and copolymers and mixtures thereof are preferred.

In addition to forming fragments that are absorbed *in vivo* ("bioresorbed"), preferred polymeric coatings for use in the methods described herein can also form an excretable and/or metabolizable fragment.

Higher order copolymers are also discussed. For example, Casey et al., U.S. Patent No. 4,438,253, which issued on March 20, 1984, discloses tri-block copolymers produced from the transesterification of poly(glycolic acid) and an hydroxyl-ended poly(alkylene glycol). Such compositions are disclosed for use as resorbable monofilament sutures. The flexibility of such compositions is controlled by the incorporation of an aromatic orthocarbonate, such as tetra-p-tolyl orthocarbonate into the copolymer structure.

Other polymers based on lactic and/or glycolic acids also described. For example, Spinu, U.S. Patent No. 5,202,413, which issued on April 13, 1993, discloses biodegradable multi-block copolymers having sequentially ordered blocks of polylactide and/or polyglycolide produced by ring-opening polymerization of lactide and/or glycolide onto either an oligomeric diol or a diamine residue followed by chain extension with a difunctional compound, such as, a diisocyanate, diacylchloride or dichlorosilane.

Bioresorbable regions of coatings useful in the described methods can be designed to be hydrolytically and/or enzymatically cleavable. For purposes of the present description, "hydrolytically cleavable" refers to the susceptibility of the copolymer, especially the bioresorbable region, to hydrolysis in water or a water-containing environment. Similarly, "enzymatically cleavable" as used herein refers to the susceptibility of the copolymer, especially the bioresorbable region, to cleavage by endo genous or exogenous enzymes.

When placed within the body, the hydrophilic region can be processed into excretable and/or metabolizable fragments. Thus, the hydrophilic region can include, for example, polyethers, polyalkylene oxides, polyols, poly(vinyl pyrrolidine), poly(vinyl alcohol), poly(alkyl oxazolines), polysaccharides, carbohydrates, peptides, proteins and copolymers and mixtures thereof. Furthermore, the hydrophilic region can also be, for example, a poly(alkylene) oxide. Such poly(alkylene) oxides can include, for example, poly(ethylene) oxide, poly(propylene) oxide and mixtures and copolymers thereof.

Polymers that are components of hydrogels are also described Hydrogels are polymeric materials that are capable of absorbing relatively large quantities of water. Examples of hydrogel forming compounds include polyacrylic acids, sodium carboxymethylcellulose, polyvinyl alcohol, polyvinyl pyrrolidine, gelatin, carrageenan and other polysaccharides, hydroxyethylenemethacrylic acid (HEMA), as well as derivatives thereof, and the like. Hydrogels can be produced that are stable, biodegradable and bioresorbable. Moreover, hydrogel compositions can include subunits that exhibit one or more of these properties.

Bio-compatible hydrogel compositions whose integrity can be controlled through crosslinking are known and are presently preferred for use in the methods described herein. For example, Hubbell *et al.,* U.S. Patent Nos. 5,410,016, which issued on April 25, 1995 and 5,529,914, which issued on June 25, 1996, disclose water-soluble systems, which are crosslinked block copolymers having a water-soluble central block segment sandwiched between two hydrolytically labile extensions. Such copolymers are further end-capped with photopolymerizable acrylate functionalities. When crosslinked, these systems become hydrogels. The water soluble central block of such copolymers can include poly(ethylene glycol); whereas, the hydrolytically labile extensions can be a poly(α-hydroxy acid), such as polyglycolic acid or polylactic acid. *See,* Sawhney et al., Macromolecules 26: 581-587 (1993).

In another embodiment, the gel is a thermoreversible gel. Thermoreversible gels including components, such as pluronics, collagen, gelatin, hyalouronic acid, polysaccharides, polyurethane hydrogel, polyurethane-urea hydrogel and combinations thereof are presently preferred.

Also disclosed is an embodiment, wherein the conjugate includes a component of a liposome. Liposomes can be prepared according to methods known to those skilled in the art, for example, as described in Eppstein et al., U.S. Patent No. 4,522,811, which issued on June 11, 1985. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its pharmaceutically acceptable salt is then introduced into the container. The container is then swirled by hand to free lipid material from the sides of the container and to disperse lipid aggregates, thereby forming the liposomal suspension.

The above-recited microparticles and methods of preparing the microparticles are offered by way of example.

The structural formats discussed above in the context of the water-soluble polymers, both straight-chain and branched are generally applicable with respect to water-insoluble polymers as well. Thus, for example, the cysteine, serine, dilysine, and trilysine branching cores can be functionalized with two water-insoluble polymer moieties. The methods used to produce these species are generally closely analogous to those used to produce the water-soluble polymers.

The *in vivo* half-life of therapeutic glycopeptides can also be enhanced with PEG moieties such as polyethylene glycol (PEG). For example, chemical modification of proteins with PEG (PEGylation) increases their molecular size and decreases their surface- and functional group-accessibility, each of which are dependent on the size of the PEG attached to the protein. This results in an improvement of plasma half-lives and in proteolytic-stability, and a decrease in immunogenicity and hepatic uptake (Chaffee et al. J. Clin. Invest. 89: 1643-1651 (1992); Pyatak et al. Res. Commun. Chem. Pathol Pharmacol. 29: 113-127 (1980)). PEGylation of interleukin-2 has been reported to increase its antitumor potency *in vivo* (Katre et al. Proc. Natl. Acad. Sci. USA. 84: 1487-1491 (1987)) and PEGylation of a F(ab')2 derived from the monoclonal antibody A7 has improved its tumor localization (Kitamura et al. Biochem. Biophys. Res. Commun. 28: 1387-1394 (1990)). Thus, in another embodiment, the *in vivo* half-life of a peptide derivatized with a PEG moiety by a method of the invention is increased relevant to the *in vivo* half-life of the non-derivatized peptide.

The increase in peptide *in vivo* half-life is best expressed as a range of percent increase in this quantity. The lower end of the range of percent increase is about 40%, about 60%, about 80%, about 100%, about 150% or about 200%. The upper end of the range is about 60%, about 80%, about 100%, about 150%, or more than about 250%.

### Preparation of Modified Sugars

In general, the sugar moiety and the modifying group are linked together through the use of reactive groups, which are typically transformed by the linking process into a new organic functional group or unreactive species. The sugar reactive functional group(s), is located at any position on the sugar moiety. Reactive groups and classes of reactions useful in practicing the present invention are generally those that are well known in the art of bioconjugate chemistry. Currently favored classes of reactions available with reactive sugar moieties are those, which proceed under relatively mild conditions. These include, but are not limited to nucleophilic substitutions (*e.g.*, reactions of amines and alcohols with acyl halides, active esters), electrophilic substitutions (*e.g*., enamine reactions) and additions to carbon-carbon and carbon-heteroatom multiple bonds (*e.g*., Michael reaction, Diels-Alder addition). These and other useful reactions are discussed in, for example, March, ADVANCED ORGANIC CHEMISTRY, 3rd Ed., John Wiley & Sons, New York, 1985; Hermanson, BIOCONJUGATE TECHNIQUES, Academic Press, San Diego, 1996; and Feeney et al., MODIFICATION OF PROTEINS; Advances in Chemistry Series, Vol. 198, American Chemical Society, Washington, D.C., 1982.

Useful reactive functional groups pendent from a sugar nucleus, linker precursor or polymeric modifying moiety precursor include, but are not limited to:
(a) carboxyl groups and various derivatives thereof including, but not limited to, N-hydroxysuccinimide esters, N-hydroxybenztriazole esters, acid halides, acyl imidazoles, thioesters, p-nitrophenyl esters, alkyl, alkenyl, alkynyl and aromatic esters;
(b) hydroxyl groups, which can be converted to, *e.g*., esters, ethers, aldehydes, *etc.*
(c) haloalkyl groups, wherein the halide can be later displaced with a nucleophilic group such as, for example, an amine, a carboxylate anion, thiol anion, carbanion, or an alkoxide ion, thereby resulting in the covalent attachment of a new group at the functional group of the halogen atom;
(d) dienophile groups, which are capable of participating in Diels-Alder reactions such as, for example, maleimido groups;
(e) aldehyde or ketone groups, such that subsequent derivatization is possible via formation of carbonyl derivatives such as, for example, imines, hydrazones, semicarbazones or oximes, or via such mechanisms as Grignard addition or alkyllithium addition;
(f) sulfonyl halide groups for subsequent reaction with amines, for example, to form sulfonamides;
(g) thiol groups, which can be, for example, converted to disulfides or reacted with acyl halides;
(h) amine or sulfhydryl groups, which can be, for example, acylated, alkylated or oxidized;
(i) alkenes, which can undergo, for example, cycloadditions, acylation, Michael addition, *etc;* and
(j) epoxides, which can react with, for example, amines and hydroxyl compounds.

The reactive functional groups can be chosen such that they do not participate in, or interfere with, the reactions necessary to assemble the reactive sugar nucleus or modifying group. Alternatively, a reactive functional group can be protected from participating in the reaction by the presence of a protecting group. Those of skill in the art understand how to protect a particular functional group such that it does not interfere with a chosen set of reaction conditions. For examples of useful protecting groups, *see,* for example, Greene et al., PROTECTIVE GROUPS IN ORGANIC SYNTHESIS, John Wiley & Sons, New York, 1991.

In the discussion that follows, a number of specific examples of modified sugars that are useful in practicing the present invention are set forth. In the exemplary embodiments, a sialic acid derivative is utilized as the sugar nucleus to which the modifying group is attached. The focus of the discussion on sialic acid derivatives is for clarity of illustration. Those of skill in the art will appreciate that a variety of other sugar moieties can be activated and derivatized in a manner analogous to that set forth using sialic acid as an example. For example, numerous methods are available for modifying galactose, glucose, N-acetylgalactosamine and fucose to name a few sugar substrates, which are readily modified by art recognized methods. *See,* for example, Elhalabi et al., Curr. Med. Chem. 6: 93 (1999); and Schafer et al., J. Org. Chem. 65: 24 (2000)).

In Scheme 1 below, the amino glycoside **1,** is treated with the active ester of a protected amino acid (*e.g*., glycine) derivative, converting the sugar amine residue into the corresponding protected amino acid amide adduct. The adduct is treated with an aldolase to form α-hydroxy carboxylate **2.** Compound **2** is converted to the corresponding CMP derivative by the action of CMP-SA synthetase, followed by catalytic hydrogenation of the CMP derivative to produce compound **3.** The amine introduced via formation of the glycine adduct is utilized as a locus of PEG or PPG attachment by reacting compound 3 with an activated (m-) PEG or (m-) PPG derivative (*e.g*., PEG-C(O)NHS, PPG-C(O)NHS), producing **4** or **5,** respectively. 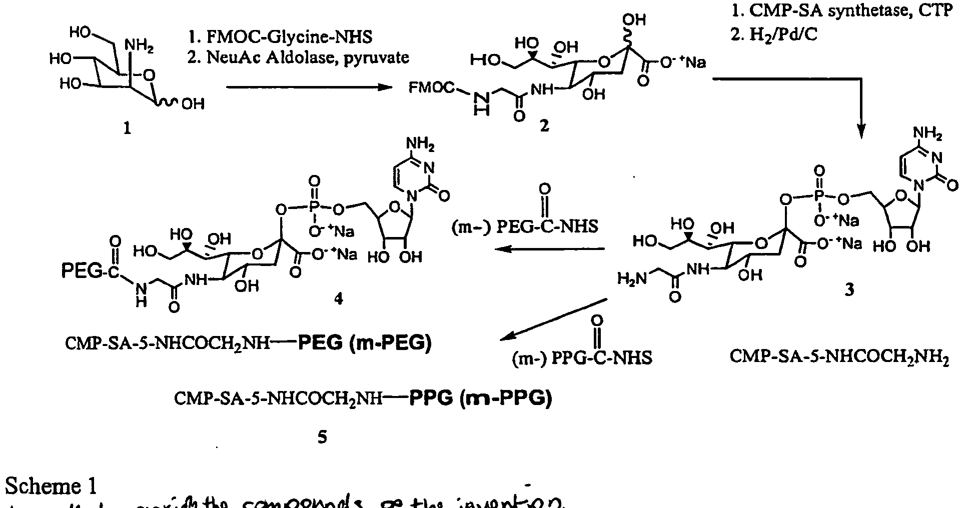 In order to provide the compounds of the invention, the polymeric modifying moiety can also be a branched moiety, such as those described herein.

An exemplary scheme for preparing the branched polymerically-modified sugars of the invention is provided below:

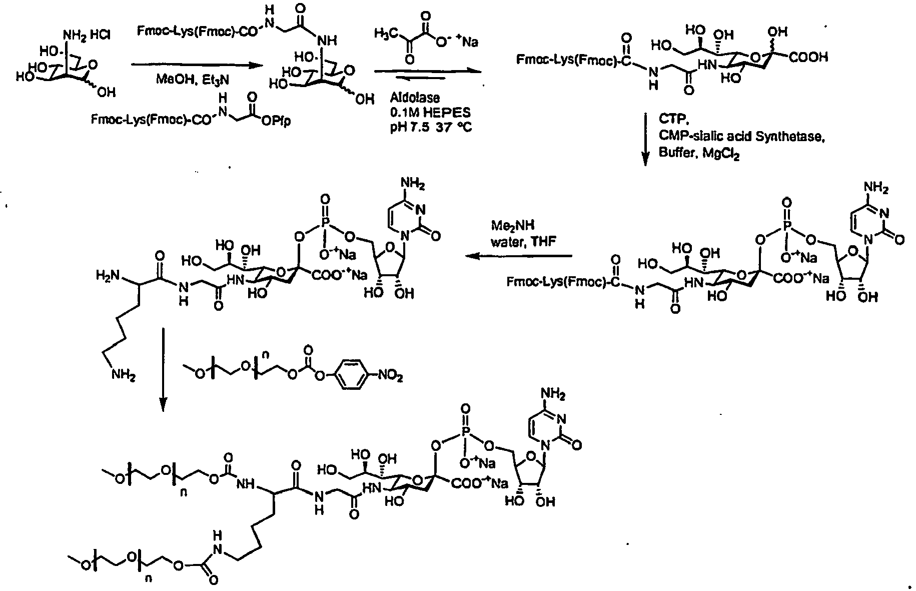

Another exemplary scheme for preparing the polymerically-modified sugars of the invention is set forth below:

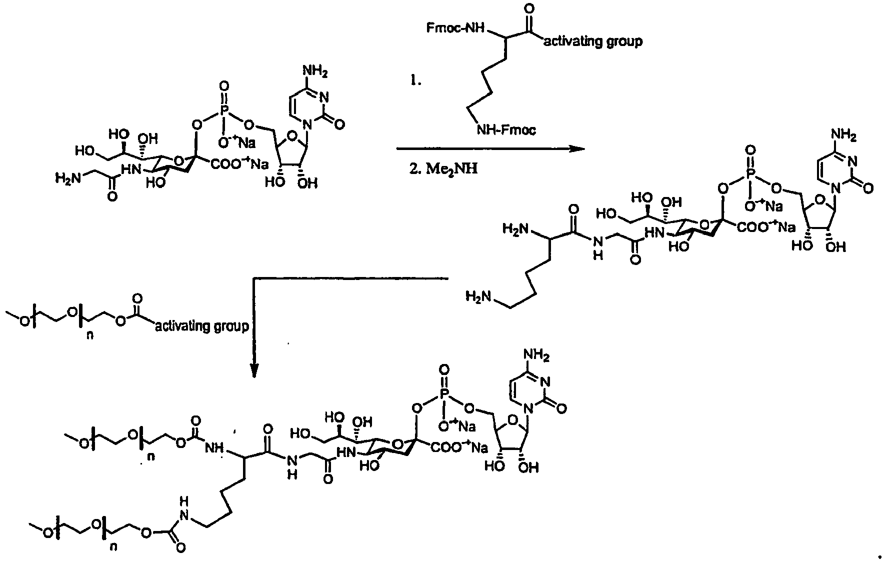

Table 1 sets forth representative examples of sugar monophosphates that are derivatized with a polymeric modifying moiety, e.g., a branched- or straight-chain PEG or PPG moiety. Certain of the compounds of Table 1 are prepared by the method of Scheme 1. Other derivatives are prepared by art-recognized methods. *See,* for example, Keppler et al., Glycobiology 11: 11R (2001); and Charter et al., Glycobiology 10: 1049 (2000)). Other amine reactive polymeric modifying moiety precursors and components, e.g., PEG analogues are commercially available, or they can be prepared by methods readily accessible to those of skill in the art.

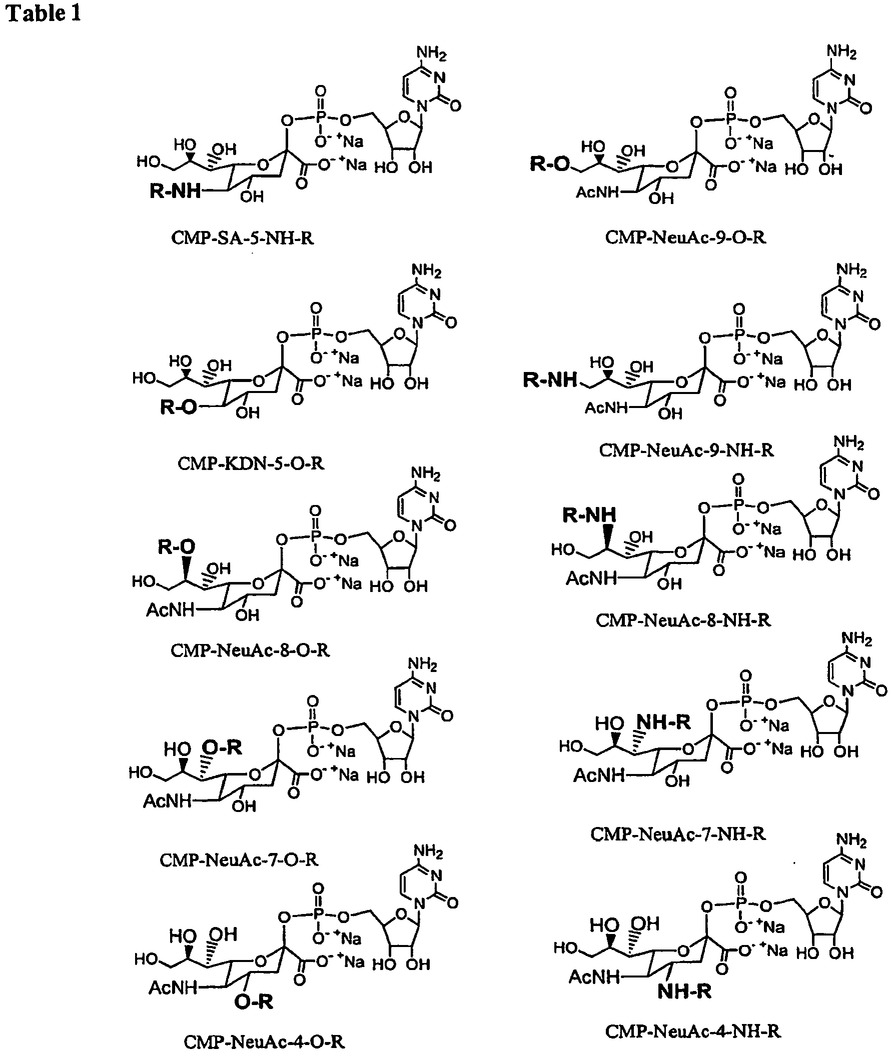

in which R is the polymeric (branched) modifying moiety.

The modified sugar phosphates of use in practicing the present invention can be substituted in other positions as well as those set forth above. Presently preferred substitutions of sialic acid are set forth in the formula below: 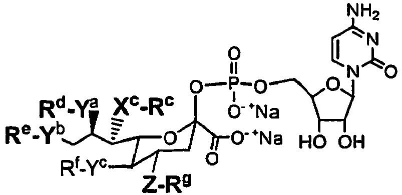 in which one or more of X^{c}, Y^{a}, Y^{b}, Y^{c} and Z is a linking group, which is preferably selected from -O-, -N(H)-, -S, CH₂-, and N(R)₂. When X^{c}, Y^{a}, Y^{b}, Y^{c} and Z is a linking group, it is attached to the polymeric modifying moiety as represented by R^{c}, R^{d}, R^{e}, R^{f} and R^{g}. Alternatively, these symbols represent a linker that is bound to a branched- or straight-chain water-soluble or water-insoluble polymer, therapeutic moiety, biomolecule or other moiety. When R^{c}, R^{d}, R^{e}, R^{f} or R^{g} is not a polymeric modifying moiety, the combination of X^{c}R^{c}, Y^{a}R^{d}, Y^{b}R^{e}, Y^{c}R^{f} or ZR^{g} is H, OH or NC(O)CH₃.

Also provided is a synthetic method for producing an activated sialic acid-polymeric modifying group conjugate that is an appropriate substrate for an enzyme that transfers the modified sugar moiety onto an acceptor, e.g., a glycosyltransferase. The method includes the steps: (a) contacting mannosamine with an activated, N-protected amino acid (or an amino acid functionalized with a polymeric modifying moiety, a linker precursor or a linker-polymeric modifying moiety cassette) under conditions appropriate to form an amide conjugate between the mannosamine and the N-protected amino acid; (b) contacting the amide conjugate with pyruvate and sialic acid aldolase under conditions appropriate to convert the amide conjugate to a sialic acid amide conjugate; (c) contacting the sialic acid amide conjugate with cytidine triphosphates, and a synthetase under conditions appropriate to form a cytidine monophosphate sialic acid amide conjugate; (d) removing the N-protecting group from the cytidine monophosphate sialic acid amide conjugate, thereby producing a free amine; and (e) contacting the free amine with an activated PEG (straight-chain or branched), thereby forming the cytidine monophosphate sialic acid-poly(ethylene glycol).

### Cross-linking Groups

Preparation of the modified sugar for use in the methods of the present invention includes attachment of a modifying group to a sugar residue and forming a stable adduct, which is a substrate for a glycosyltransferase. The sugar and modifying group can be coupled by a zero- or higher-order cross-linking agent. Exemplary bifunctional compounds which can be used for attaching modifying groups to carbohydrate moieties include, but are not limited to, bifunctional poly(ethyleneglycols), polyamides, polyethers, polyesters and the like. General approaches for linking carbohydrates to other molecules are known in the literature. *See,* for example, Lee et al., Biochemistry 28: 1856 (1989); Bhatia et al., Anal. Biochem. 178: 408 (1989); Janda et al., J. Am. Chem. Soc. 112: 8886 (1990) and Bednarski et al., WO 92/18135. In the discussion that follows, the reactive groups are treated as benign on the sugar moiety of the nascent modified sugar. The focus of the discussion is for clarity of illustration. Those of skill in the art will appreciate that the discussion is relevant to reactive groups on the modifying group as well.

An exemplary strategy involves incorporation of a protected sulfhydryl onto the sugar using the heterobifunctional crosslinker SPDP (n-succinimidyl-3-(2-pyridyldithio)propionate and then deprotecting the sulfhydryl for formation of a disulfide bond with another sulfhydryl on the modifying group.

A variety of reagents are used to modify the components of the modified sugar with intramolecular chemical crosslinks (for reviews of crosslinking reagents and crosslinking procedures see: Wold, F., Meth. Enzymol. 25: 623-651, 1972; Weetall, H. H., and Cooney, D. A., In: ENZYMES AS DRUGS. (Holcenberg, and Roberts, eds.) pp. 395-442, Wiley, New York, 1981; Ji, T. H., Meth. Enzymol. 91: 580-609, 1983; Mattson et al., Mol. Biol. Rep. 17: 167-183, 1993, all of which are incorporated herein by reference). Preferred crosslinking reagents are derived from various zero-length, homo-bifunctional, and hetero-bifunctional crosslinking reagents. Zero-length crosslinking reagents include direct conjugation of two intrinsic chemical groups with no introduction of extrinsic material.

### Conjugation of Modified Sugars to Peptides

The modified sugars are conjugated to a glycosylated or non-glycosylated peptide using an appropriate enzyme to mediate the conjugation. Thus, the compounds of the invention, particularly the nucleotide sugars are preferably substrates for enzymes that transfer sugar moieties from a nucleotide sugar onto an amino acid, glycosyl, or aglycone acceptor moiety. Nucleotide sugars that act as sugar donors for acceptors, e.g., galactosyl acceptors, e.g., GalNAc, Galβ1,4GlcNAc, Galβ1,4GalNAc, Galβ1,3GalNAc, lacto-N-tetraose, Galβ1,3GlcNAc, Galβ1,3Ara, Galβ1,6GlcNAc, Galβ1,4Glc (lactose), and other acceptors well known to those of skill in the art (*see, e.g.,* Paulson et al., J. Biol. Chem. 253: 5617-5624 (1978)).

Exemplary enzymes for which the modified nucleotide sugars of the invention are substrates include glycosyltransferases. The glycosyltransferase can be cloned, or isolated from any source. Many cloned glycosyltransferases are known, as are their polynucleotide sequences. *See, e.g.,* "The WWW Guide To Cloned Glycosyltransferases," (http://www.vei.co.uk/TGN/gt guide.htm). Glycosyltransferase amino acid sequences and nucleotide sequences encoding glycosyltransferases from which the amino acid sequences can be deduced are also found in various publicly available databases, including GenBank, Swiss-Prot, EMBL, and others.

Glycosyltransferases for which the compounds of the invention are substrates include, but are not limited to, galactosyltransferases, fucosyltransferases, glucosyltransferases, N-acetylgalactosaminyltransferases, N-acetylglucosaminyltransferases, glucuronyltransferases, sialyltransferases, mannosyltransferases, glucuronic acid transferases, galacturonic acid transferases, and oligosaccharyltransferases. Suitable glycosyltransferases include those obtained from eukaryotes, as well as from prokaryotes.

In some embodiments, the compound of the invention is a substrate for a fucosyltransferase. Fucosyltransferases are generally known to those of skill in the art, and are exemplified by enzymes that transfer L-fucose from GDP-fucose to a hydroxy position of an acceptor sugar.

In another group of embodiments, the compound is a substrate for a galactosyltransferase. Exemplary galactosyltransferases include α(1,3) galactosyltransferases (E.C. No. 2.4.1.151, *see, e.g.,* Dabkowski et al., Transplant Proc. 25:2921 (1993) and Yamamoto et al. Nature 345: 229-233 (1990), bovine (GenBank j04989, Joziasse et al., J. Biol. Chem. 264: 14290-14297 (1989)), murine (GenBank m26925; Larsen et al., Proc. Nat'l. Acad. Sci. USA 86: 8227-8231 (1989)), porcine (GenBank L36152; Strahan et al., Immunogenetics 41: 101-105 (1995)). Another suitable α1,3 galactosyltransferase is that which is involved in synthesis of the blood group B antigen (EC 2.4.1.37, Yamamoto et al., J. Biol. Chem. 265: 1146-1151 (1990) (human)). Yet a further exemplary galactosyltransferase is core Gal-T1. Still further examples include β(1,4) galactosyltransferases, which include, for example, EC 2.4.1.90 (LacNAc synthetase) and EC 2.4.1.22 (lactose synthetase) (bovine (D'Agostaro et al., Eur. J. Biochem. 183: 211-217 (1989)), human (Masri et al., Biochem. Biophys. Res. Commun. 157: 657-663 (1988)), murine (Nakazawa et al., J. Biochem.104: 165-168 (1988)), as well as E.C. 2.4.1.38 and the ceramide galactosyltransferase (EC 2.4.1.45, Stahl et al., J. Neurosci. Res. 38: 234-242 (1994)). Other suitable galactosyltransferases include, for example, α1,2 galactosyltransferases (from e.g., *Schizosaccharomyces pombe,* Chapell et al., Mol. Biol. Cell 5: 519-528 (1994)). Also suitable in the practice of the invention are soluble forms of α1, 3- galactosyltransferase such as that reported by Cho et al., J. Biol. Chem., 272: 13622-13628 (1997).

### a) Sialyltransferases

Sialyltransferases are another type of glycosyltransferase for which the compounds of the invention are substrates. Examples include ST3Ga1 III (*e.g*., a rat or human ST3Ga1 III), ST3Ga1 IV, ST3Ga1 I, ST6Ga1 I, ST3Ga1 V, ST6Ga1 II, ST6GalNAc I, ST6GalNAc II, and ST6GalNAc III (the sialyltransferase nomenclature used herein is as described in Tsuji et al., Glycobiology 6: v-xiv (1996)). An exemplary α(2,3)sialyltransferase referred to as α(2,3)sialyltransferase (EC 2.4.99.6) transfers sialic acid to the non-reducing terminal Gal of a Galβ1→3Glc disaccharide or glycoside. *See,* Van den Eijnden et al., J. Biol. Chem. 256: 3159 (1981), Weinstein et al., J. Biol. Chem. 257: 13845 (1982) and Wen et al., J. Biol. Chem. 267: 21011 (1992). Another exemplary α2,3-sialyltransferase (EC 2.4.99.4) transfers sialic acid to the non-reducing terminal Gal of the disaccharide or glycoside. *see,* Rearick et al., J. Biol. Chem. 254: 4444 (1979) and Gillespie et al., J. Biol. Chem. 267: 21004 (1992). Further exemplary enzymes include Gal-β-1,4-GlcNAc α-2,6 sialyltransferase (*See,* Kurosawa et al. Eur. J. Biochem. 219: 375-381 (1994)). Other sialyltransferases for which the compounds of the invention are substrates include those that form polysialic acids. Examples include the α-2,8-polysialyltransferases, e.g., ST8SiaI, ST8SiaII, ST8SiaIII, STBSiaN and ST8SiaV. See for example, Angata et al. J. Biol. Chem. 275: 18594-18601 (2000); Kono et al., J. Biol. Chem. 271: 29366-29371 (1996); Greiner et al., Infect. Immun. 72: 4249-4260 (2004); and Jones et al., J. Biol. Chem. 277: 14598-14611 (2002).

An example of a sialyltransferase that is useful in the claimed methods is ST3Ga1 III, which is also referred to as α(2,3)sialyltransferase (EC 2.4.99.6). This enzyme catalyzes the transfer of sialic acid to the Gal of a Galβ1,3GlcNAc or Galβ1,4GlcNAc glycoside (*see, e.g*., Wen et al., J. Biol. Chem. 267: 21011 (1992); Van den Eijnden et al., J. Biol. Chem. 256: 3159 (1991)). Still further sialyltransferases include those isolated from Campylobacter jejuni, including the α(2,3). *See,* e.g, WO99/49051.

Preferably, the compounds of the invention are substrates for an enzyme that transfers the modifies sialic acid to the sequence Galβ1,4GlcNAc-, the most common penultimate sequence underlying the terminal sialic acid on fully sialylated carbohydrate structures.

### b) GalNAc transferases

Selected compounds of the invention are substrates for N-acetylgalactosaminyltransferases. Exemplary N-acetylgalactosaminyltransferases include, but are not limited to, α(1,3) N-acetylgalactosaminyltransferase, β(1,4) N-acetylgalactosaminyltransferases (Nagata et al., J. Biol. Chem. 267: 12082-12089 (1992) and Smith et al., J. Biol Chem. 269: 15162 (1994)) and polypeptide N-acetylgalactosaminyltransferase (Homa et al., J. Biol. Chem. 268: 12609 (1993)).

### c) Glycosidases

This invention also encompasses substrates for wild-type and mutant glycosidases. Mutant β-galactosidase enzymes have been demonstrated to catalyze the formation of disaccharides through the coupling of α-glycosyl fluoride to a galactosyl acceptor molecule. (Withers, U.S. Pat. No. 6,284,494; issued Sept. 4, 2001). Other glycosidases of use in this invention include, for example, β-glucosidases, β-galactosidases, β-mannosidases, β-acetyl glucosaminidases, β-N-acetyl galactosaminidases, β-xylosidases, β-fucosidases, cellulases, xylanases, galactanases, mannanases, hemicellulases, amylases, glucoamylases, α-glucosidases, α-galactosidases, α-mannosidases, α-N-acetyl glucosaminidases, α-N-acetyl galactose-aminidases, α-xylosidases, α-fucosidases, and neuraminidases/sialidases, endoglycoceramidases.

The following examples are provided to illustrate selected embodiments of the invention

### EXAMPLES

### Example 1

### Preparation of UDP-GalNAc-6'-CHO

UDP-GalNAc (200 mg, 0.30 mmoles) was dissolved in a 1 mM CuSO₄ solution (20 mL) and a 25 mM NaH₂PO₄ solution (pH 6.0; 20 mL). Galactose oxidase (240 U; 240 µL) and catalase (13000 U; 130 µL) were then added, the reaction system equipped with a balloon filled with oxygen and stirred at room temperature for seven days. The reaction mixture was then filtered (spin cartridge; MWCO 5K) and the filtrate (-40 mL) was stored at 4° C until required. TLC (silica; EtOH/water (7/2); R_{f} = 0.77; visualized with anisaldehyde stain).

### Example 2

### Preparation of UDP-GalNAc-6'-NH₂):

Ammonium acetate (15 mg, 0.194 mmoles) and NaBH₃CN (1M THF solution; 0.17 mL, 0.17 mmoles) were added to the UDP-GalNAc-6' -CHO solution from above (2 mL or ~ 20 mg) at 0°C and allowed to warm to room temperature overnight. The reaction was filtered through a G-10 column with water and the product collected. The appropriate fractions were freeze-dried and stored frozen. TLC (silica; ethanol/water (7/2); R_{f} = 0.72; visualized with ninhydrin reagent).

### Example 3

### Preparation of UDP-GalNAc-6-NHCO(CH₂)₂-O-PEG-OMe (1 KDa).

The galactosaminyl-1-phosphate-2-NHCO(CH₂)₂-O-PEG-OMe (1 KDa) (58 mg, 0.045 mmoles) was dissolved in DMF (6 mL) and pyridine (1.2 mL). UMP-morpholidate (60 mg, 0.15 mmoles) was then added and the resulting mixture stirred at 70°C for 48 h. The solvent was removed by bubbling nitrogen through the reaction mixture and the residue purified by reversed phase chromatography (C-18 silica, step gradient between 10 to 80%, methanol/water). The desired fractions were collected and dried at reduced pressure to yield a white solid. TLC (silica, propanol/H₂O/NH₄OH, (30/20/2), R_{f}= 0.54). MS (MALDI): Observed, 1485, 1529, 1618, 1706.

### Example 4

### Preparation of Cysteine-PEG₂ (2)

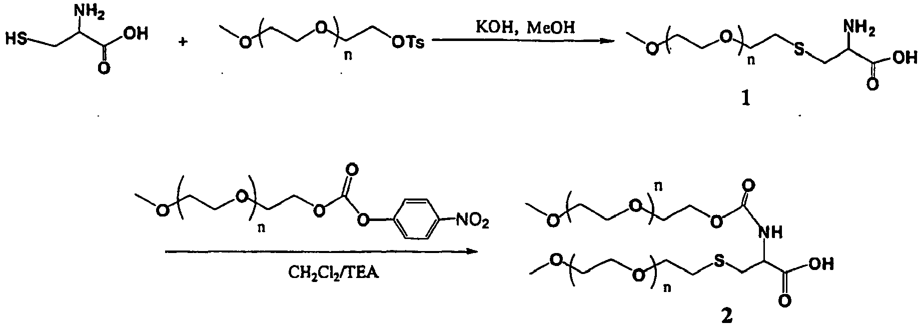

### 4.1 Synthesis of Compound 1

Potassium hydroxide (84.2 mg, 1.5 mmol, as a powder) was added to a solution of L-cysteine (93.7mg, 0.75 mmol) in anhydrous methanol (20L) under argon. The mixture was stirred at room temperature for 30 min, and then mPEG-O-tosylate of molecular mass 20 kilodalton (Ts; 1.0 g, 0.05 mmol) was added in several portions over 2 hours. The mixture was stirred at room temperature for 5 days, and concentrated by rotary evaporation. The residue was diluted with water (30 mL), and stirred at room temperature for 2 hours to destroy any excess 20 kilodalton mPEG- O-tosylate. The solution was then neutralized with acetic acid, the pH adjusted to pH 5.0 and loaded onto a reversed phase chromatography (C-18 silica) column. The column was eluted with a gradient of methanol/water (the product elutes at about 70% methanol), product elution monitored by evaporative light scattering, and the appropriate fractions collected and diluted with water (500 mL). This solution was chromatographed (ion exchange, XK 50 Q, BIG Beads, 300 ml, hydroxide form; gradient of water to water/acetic acid-0.75N) and the pH of the appropriate fractions lowered to 6.0 with acetic acid. This solution was then captured on a reversed phase column (C-18 silica) and eluted with a gradient of methanol/water as described above. The product fractions were pooled, concentrated, redissolved in water and freeze-dried to afford a white solid (1). Structural data for the compound were as follows: ¹H-NMR (500 MHz; D₂O) δ 2.83 (t, 2H, O-C-CH₂-S), 3.05 (q, 1H, S-CHH-CHN), 3.18 (q, 1H, (q, 1H, S-CHH-CHN), 3.38 (s, 3H, CH₃O), 3.7 (t, OCH₂CH₂O), 3.95 (q, 1H, CHN). The purity of the product was confirmed by SDS PAGE.

### 4.2 Synthesis of Compound 2 (Cysteine-PEG₂)

Triethylamine (~0.5 mL) was added dropwise to a solution of compound 1 (440 mg, 22 µmol) dissolved in anhydrous CH₂Cl₂ (30 mL) until the solution was basic. A solution of 20 kilodalton mPEG-O-p-nitrophenyl carbonate (660 mg, 33 µmol) and N-hydroxysuccinimide (3.6 mg, 30.8 µmol) in CH₂Cl₂ (20 mL) was added in several portions over 1 hour at room temperature. The reaction mixture was stirred at room temperature for 24 hours. The solvent was then removed by rotary evaporation, the residue was dissolved in water (100 mL), and the pH adjusted to 9.5 with 1.0 N NaOH. The basic solution was stirred at room temperature for 2 hours and was then neutralized with acetic acid to a pH 7.0. The solution was then loaded onto a reversed phase chromatography (C-18 silica) column. The column was eluted with a gradient of methanol/water (the product elutes at about 70% methanol), product elution monitored by evaporative light scattering, and the appropriate fractions collected and diluted with water (500 mL). This solution was chromatographed (ion exchange, XK 50 Q, BIG Beads, 300 mL, hydroxide form; gradient of water to water/acetic acid-0.75N) and the pH of the appropriate fractions lowered to 6.0 with acetic acid. This solution was then captured on a reversed phase column (C-18 silica) and.eluted with a gradient of methanol/water as described above. The product fractions were pooled, concentrated, redissolved in water and freeze-dried to afford a white solid (2). Structural data for the compound were as follows: ¹H-NMR (500 MHz; D₂O) δ 2.83 (t, 2H, O-C-CH₂-S), 2.95 (t, 2H, O-C-CH₂-S), 3.12 (q, 1H, S-CHH-CHN), 3.39 (s, 3H CH₃O), 3.71 (t, OCH₂CH₂O). The purity of the product was confirmed by SDS PAGE.

### Example 5

### Preparation of UDP-GalNAc-6-NHCO(CH₂)₂-O-PEG-OMe (1 KDa).

Galactosaminyl-1-phosphate-2-NHCO(CH₂)₂-O-PEG-OMe (1 kilodalton) (58 mg, 0.045 mmoles) was dissolved in DMF (6 mL) and pyridine (1.2 mL). UMP-morpholidate (60 mg, 0.15 mmoles) was then added and the resulting mixture stirred at 70°C for 48 h. The solvent was removed by bubbling nitrogen through the reaction mixture and the residue purified by reversed phase chromatography (C-18 silica, step gradient between 10 to 80%, methanol/water). The desired fractions were collected and dried at reduced pressure to yield a white solid. TLC (silica, propanol/H₂O/NH₄OH, (30/20/2), R_{f} = 0.54). MS (MALDI): Observed, 1485,1529, 1618, 1706.

### SDS PAGE Procedure

The purity of the products, **1** and **2**, were confirmed by SDS PAGE. A 4-20% Tris-Glycine SDS PAGE gel (Invitrogen) was used. The sample was mixed 1:1 with SDS Sample Buffer, and was run in Tris-Glycine Running Buffer (LC2675-5) at a constant voltage (125 V) for 1 hr 50 min. After electrophoresis, the gel was washed with water (100 mL) for 10 min followed by a wash with a 5% barium chloride aqueous solution (100 mL) for 10 min. Products **1** or **2** were visualized by staining the gels with 0.1 N iodine solution (4.0 mL) at room temperature and the staining process stopped by washing the gels with water. The visualized product bands were scanned with an HP Scanjet 7400C, and the image of the gel was optimized with the HP Precision Scan Program.

## Claims

1. A compound having a formula that is a member selected from: 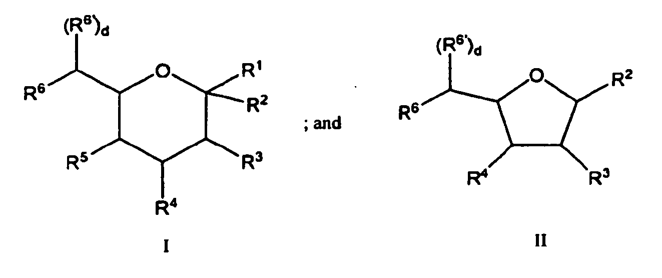 wherein
R¹ is H, CH₂OR⁷, COOR⁷ or OR⁷
in which
d is 0 or 1;
R⁷ represents H, substituted or unsubstituted alkyl or substituted or unsubstituted heteroalkyl;
R² is a member selected from H, OH, an activating group and a moiety that includes a nucleotide;
R³, R⁴, R⁵, R⁶ and R^{6'} are independently selected from H, substituted or unsubstituted alkyl, OR⁹, and NHC(O)R¹⁰
wherein
R⁹ and R¹⁰ are independently selected from H, substituted or unsubstituted alkyl or substituted or unsubstituted heteroalkyl,
at least one of R³, R⁴, R⁵, R⁶ and R^{6'} includes a moiety having the formula: 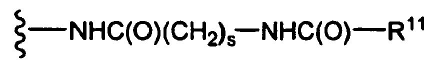 wherein
s is an integer from 0 to 20; and
R¹¹ is a polymeric modifying moiety having the formula: 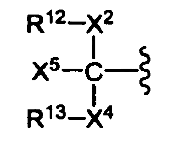 wherein
X² and X⁴ are independently selected from S, SC(O)NH,
HNC(O)S, SC(O)O, O, NH, NHC(O), (O)CNH and NHC(O)O, OC(O)NH and (CH₂)_{g}Y",
wherein
g is an integer from 1 to 50; and
Y" is a member selected from O, S and NH;
X⁵ is a non-reactive group; and
R¹² and R¹³ are independently selected polymeric arms, , wherein said polymeric modifying moiety has a formula selected from: 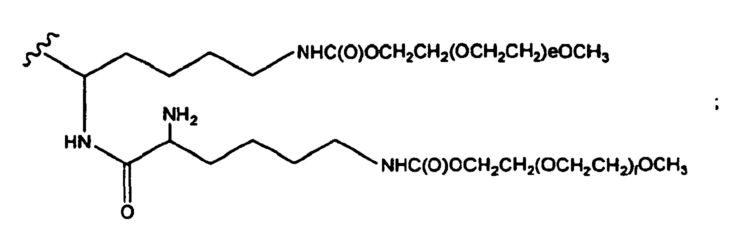 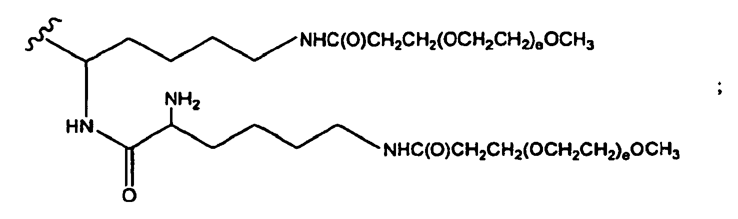 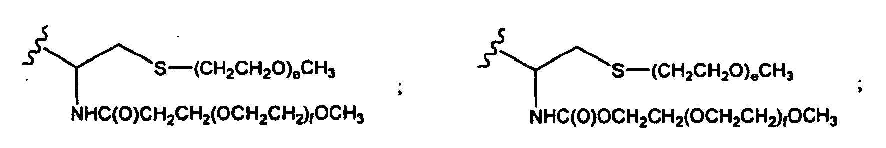 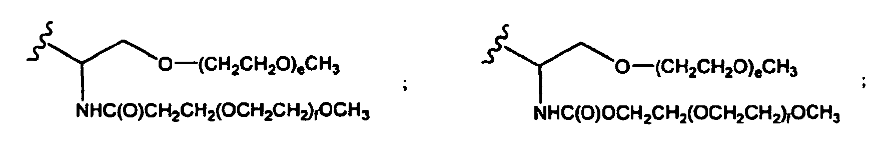 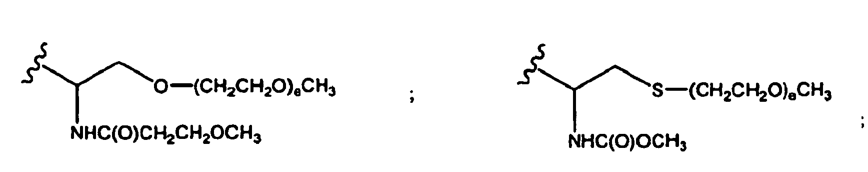 and 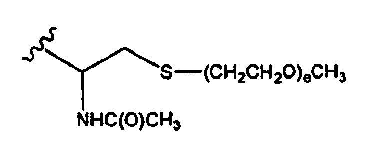 wherein e and f are independently selected from 1 to 2500.

2. The compound according to claim 1 wherein R² has the formula: 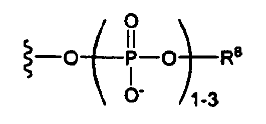 in which R⁸ is a nucleoside.

3. The compound according to claim 2 wherein R⁸ is a member selected from cytidine, uridine, guanosine, adenosine and thymidine.

4. (Amended) The compound according to any of the foregoing claims having the formula: 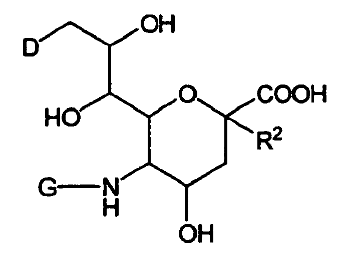 in which
D is a member selected from -OH and (R¹¹)_{w'}-L-;
G represents is a member selected from (R¹¹)_{w'}-L- and -C(O)(C₁-C₆)alkyl;
w' is an integer from 2 to 6, and
at least one of D and G is (R¹¹)_{w'}-L-,
wherein
L is a member selected from a bond and a linking group selected from substituted or unsubstituted alkyl and substituted or unsubstituted heteroalkyl moieties.

5. The compound according to any of the foregoing claims wherein said compound is a substrate for an enzyme that transfers a sugar moiety from a member selected from an activated sugar, a nucleotide sugar and combinations thereof onto an acceptor moiety of a substrate.

6. The compound according to claim 5 wherein said acceptor moiety is a member selected from a glycosyl residue, an amino acid residue and an aglycone.

7. The compound according to any of the foregoing claims, having the formula: 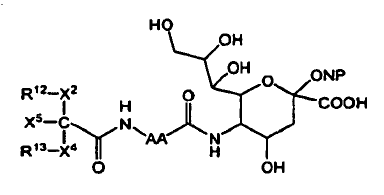 wherein
AA is -CH₂-; and
NP is a nucleotide phosphate.

## Patentansprüche

1. Eine Verbindung mit einer Formel, die ein Mitglied ausgewählt aus: 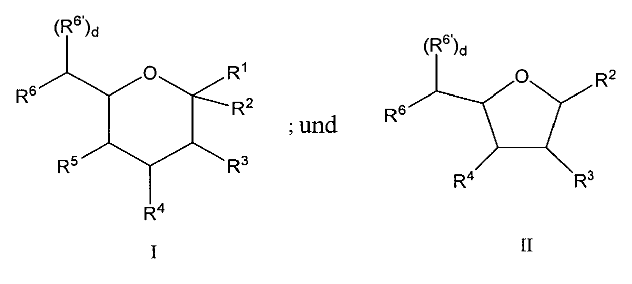 ist, wobei
R¹ H, CH₂OR⁷, COOR⁷ oder OR⁷ ist
d 0 oder 1 ist;
R⁷ H, ein substitutiertes oder unsubstitutiertes Alkyl or ein substitutiertes oder unsubstitutiertes Heteroalkyl repräsentiert;
R² ein Mitglied ausgewählt aus H, OH, einer aktivierenden Gruppe und einem Rest, der ein Nukleotid beinhaltet, ist;
R³, R⁴, R⁵, R⁶ und R^{6'} unabhängig voneinander ausgewählt sind aus H, einem substitutierten oder unsubstitutierten Alkyl, OR⁹ und NHC(O)R¹⁰,
wobei
R⁹ und R¹⁰ unabhängig voneinander ausgewählt sind aus H, einem substitutierten oder unsubstitutierten Alkyl oder einem substitutierten oder unsubstitutierten Heteroalkyl,
mindestens eines von R³, R⁴, R⁵, R⁶ und R^{6'} einen Rest mit der Formel: 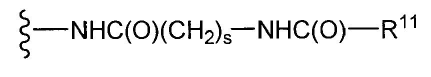 beinhaltet, wobei
s eine ganze Zahl von 0 bis 20 ist; und
R¹¹ ein modifizierender Polymerrest mit der Formel: 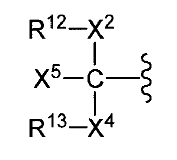 ist, wobei
X² und X⁴ unabhängig voneinander ausgewählt sind aus S, SC(O)NH, HNC(O)S, SC(O)O, O, NH, NHC(O), (O)CNH und NHC(O)O, OC(O)NH und (CH₂)_{g}Y",
wobei
g eine ganze Zahl von 1 bis 50 ist; und Y" ein Mitglied ausgewählt aus O, S und NH ist;
X⁵ eine nicht-reaktive Gruppe ist; und
R¹² und R¹³ unabhängig voneinander ausgewählte Polymerarme sind, wobei der modifizierende Polymerrest eine Formel hat, die ausgewählt ist aus: 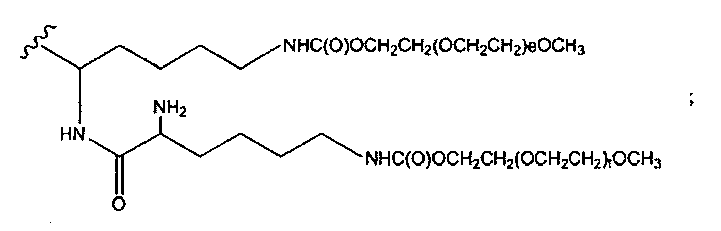 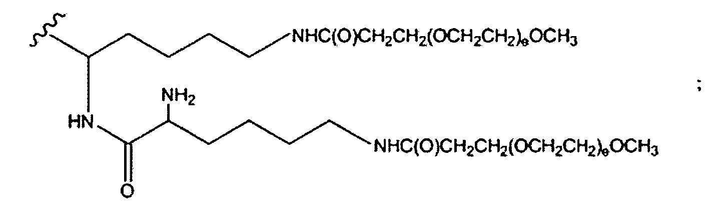 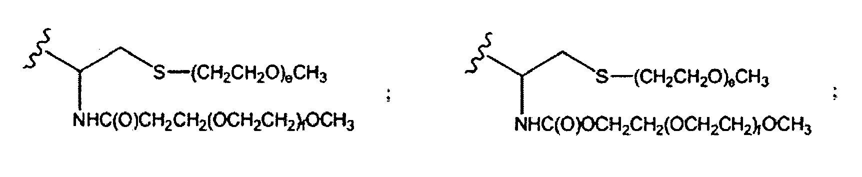 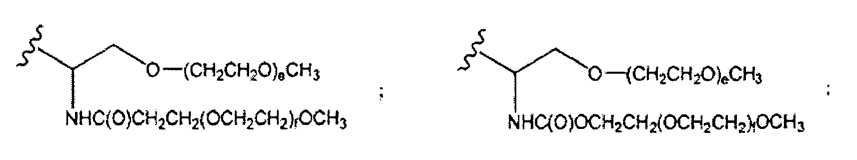 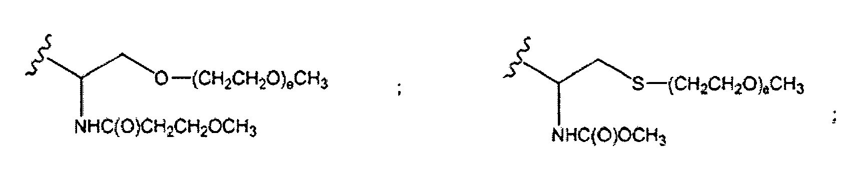 und 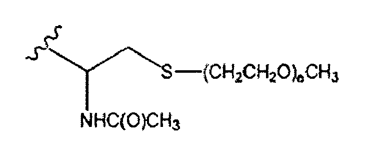 wobei e und f unabhängig voneinander aus 1 bis 2500 ausgewählt sind.

2. Die Verbindung nach Anspruch 1, wobei R² die Formel: 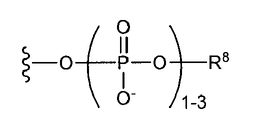 hat, in der R⁸ ein Nukleosid ist.

3. Die Verbindung nach Anspruch 2, wobei R⁸ ein Mitglied ausgewählt aus Cytidin, Uridin, Guanosin, Adenosin und Thymidin ist.

4. Die Verbindung nach einem der vorangehenden Ansprüche mit der Formel: 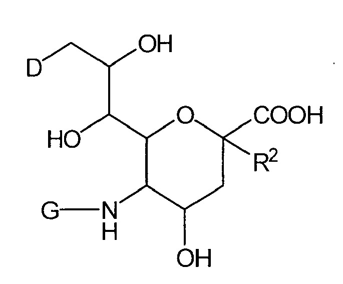 in der
D ein Mitglied ausgewählt aus -OH und (R¹¹)_{w'}-L- ist;
G ein Mitglied ausgewählt aus (R¹¹)_{w'}-L- und -C(O)(C₁-C₆)-Alkyl repräsentiert;
w' eine ganze Zahl von 2 bis 6 ist, und
mindestens eines von D und G (R¹¹)_{w'}-L- ist,
wobei
L ein Mitglied ausgewählt aus einer Bindung und einer Verbindungsgruppe ist, die aus substitutierten oder unsubstitutierten Alkyl- und substitutierten oder unsubstitutierten Heteroalkylresten ausgewählt ist.

5. Die Verbindung nach einem der vorangehenden Ansprüche, wobei die Verbindung ein Substrat für ein Enzym ist, das einen Zuckerrest von einem Mitglied ausgewählt aus einem aktivierten Zucker, einem Nukleotidzucker und Kombinationen davon auf einen Akzeptorrest eines Substrats transferiert.

6. Die Verbindung nach Anspruch 5, wobei der Akzeptorrest ein Mitglied ausgewählt aus einem Glycosylrest, einem Aminosäurerest und einem Aglycon ist.

7. Die Verbindung nach einem der vorangehenden Ansprüche mit der Formel: 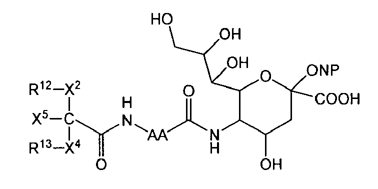 wobei
AA-CH₂- ist; und
NP ein Nukleotidphosphat ist.

## Revendications

1. Composé ayant une formule qui est un membre choisi parmi : 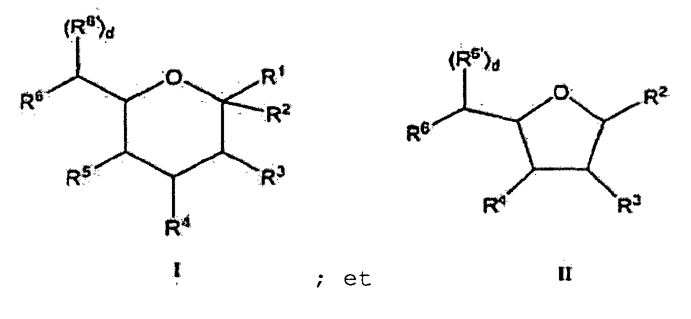 dans lequel
R¹ est H, CH₂OR⁷, COOR⁷ ou OR⁷
où
d est 0 ou 1 ;
R⁷ représente H, un groupe alkyle substitué ou non substitué ou un groupe hétéroalkyle substitué ou non substitué ;
R² est un élément choisi parmi H, OH, un groupe d'activation ou un fragment qui comprend un nucléotide ;
R³, R⁴, R⁵, R⁶ et R^{6'} sont choisis indépendamment parmi H, un groupe alkyle substitué ou non substitué, OR⁹ et NHC(O)R¹⁰⁻,
où
R⁹ et R¹⁰ sont choisis indépendamment parmi H, un groupe alkyle substitué ou non substitué ou hétéroalkyle substitué ou non substitué,
au moins l'un des R³, R⁴, R⁵, R⁶ et R^{6'} comprend un fragment de formule 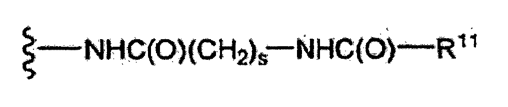 où
s est un nombre entier de 0 à 20 ; et
R¹¹ est un fragment de modification polymère ayant la formule : 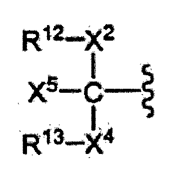 où
X² et X⁴ sont choisis indépendamment parmi S, SC(O)NH, HNC(O)S, SC(O)O, O, NH, NHC(O), (O)CNH et NHC(O)O, OC(O)NH et (CH₂)_{g}Y",
où
g est un nombre entier de 1 à 50 ; et
Y" est un membre choisi parmi O, S et NH ;
X⁵ est un groupe non réactif ; et
R¹² et R¹³ sont des bras polymères choisis indépendamment,
dans lequel ledit fragment de modification polymère ayant une formule choisie parmi : 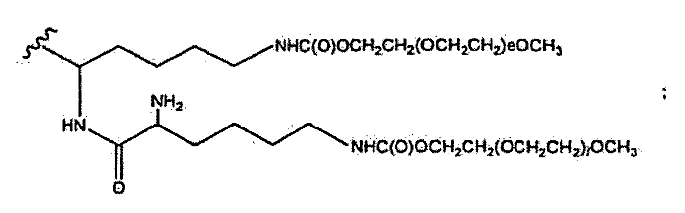 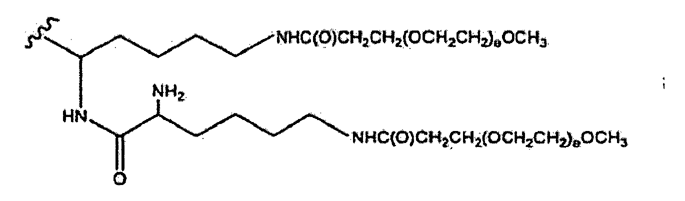 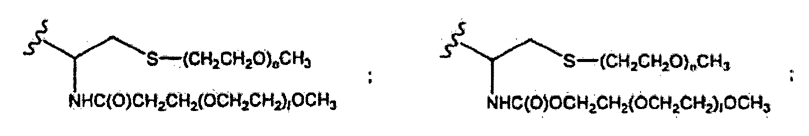 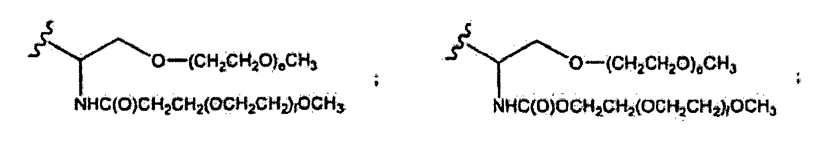 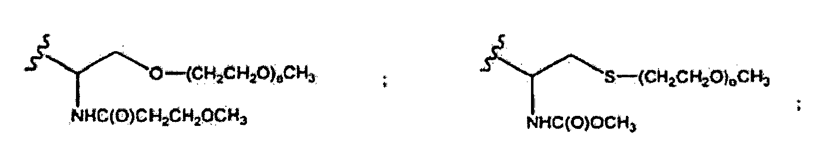 et 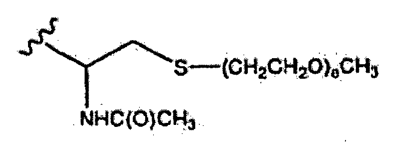 où e et f sont choisis indépendamment de 1 à 2500.

2. Composé selon la revendication 1, dans lequel R² présente la formule : 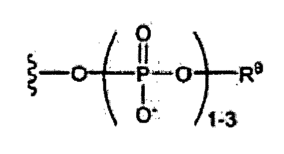 où R⁸ est un nucléoside.

3. Composé selon la revendication 2, dans lequel R⁸ est un membre choisi parmi la cytidine, l'uridine, la guanosine, l'adénosine et la thymidine.

4. Composé selon l'une quelconque des revendications précédentes, ayant la formule: 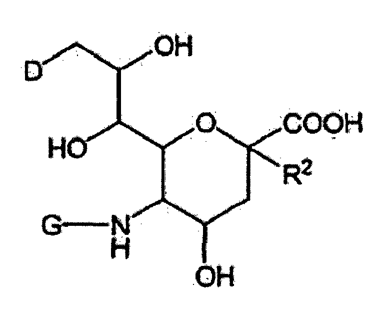 où
D est un membre choisi parmi -OH et (R¹¹)_{w'}-L- ;
G représente un membre choisi parmi (R¹¹)_{w'}-L- et - C(O)alkyle (en C₁ à C₆) ;
w' est un nombre entier de 2 à 6, et
au moins l'un des éléments D et G est (R¹¹)_{w'}-L-,
où
L est un membre choisi parmi une liaison et un groupe de liaison choisi parmi les fragments alkyle substitués ou non substitués et hétéroalkyle substitués ou non substitués.

5. Composé selon l'une quelconque des revendications précédentes, dans lequel ledit composé est un substrat d'une enzyme qui transfère un fragment de sucre d'un membre choisi parmi un sucre activé, un sucre de nucléotide et des combinaisons de ceux-ci sur un fragment accepteur d'un substrat.

6. Composé selon la revendication 5, dans lequel ledit fragment accepteur est un membre choisi parmi un résidu de glycosyle, un résidu d'acide aminé et un aglycone.

7. Composé selon l'une quelconque des revendications précédentes, ayant la formule : 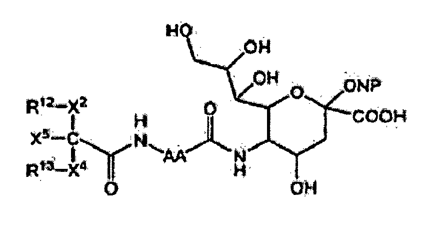 où
AA est -CH₂- ; et
NP est un phosphate de nucléotide.
